(19) Europäisches Patentamt
European Patent Office
Office européen des brevets

(11) EP 1 421 191 B1

(12) **EUROPEAN PATENT SPECIFICATION**

(45) Date of publication and mention of the grant of the patent:
**28.09.2011 Bulletin 2011/39**

(21) Application number: **02711860.3**

(22) Date of filing: **11.02.2002**

(51) Int Cl.:
***C12N 15/13*** (2006.01) ***C07K 16/28*** (2006.01)
***C12N 15/79*** (2006.01) ***C12N 15/10*** (2006.01)
***A61K 39/395*** (2006.01) ***A61P 37/00*** (2006.01)

(86) International application number:
**PCT/EP2002/001420**

(87) International publication number:
**WO 2002/072832 (19.09.2002 Gazette 2002/38)**

(54) **Humanised antibodies specific for both CD45RB and CD45RO**

Humanisierte Antikörper spezifisch für sowohl CD45RB als auch CD45RO

Anticorps humanisés spécifiques pour à la fois CD45RB et CD45RO

(84) Designated Contracting States:
**AT BE CH CY DE DK ES FI FR GB GR IE IT LI LU MC NL PT SE TR**

(30) Priority: **12.02.2001 GB 0103389**

(43) Date of publication of application:
**26.05.2004 Bulletin 2004/22**

(73) Proprietor: **Novartis AG**
**4056 Basel (CH)**

(72) Inventors:
- **AVERSA, Gregorio**
  **1130 Vienna (AT)**
- **KOLBINGER, Frank**
  **79395 Neuenburg (DE)**
- **CARBALLIDO HERRERA, José, M.**
  **1235 Vienna (AT)**
- **ASZÓDI, András**
  **1140 Vienna (AT)**
- **SALDANHA, José, W.**
  **London EN1 3BD (GB)**
- **HALL, Bruce, M.**
  **Strathfield, NSW 2135 (AU)**

(74) Representative: **de Weerd, Petrus G.W. et al**
**Novartis International AG**
**Corporate Intellectual Property**
**4002 Basel (CH)**

(56) References cited:
**WO-A-98/11918**

- **G. AVERSA ET AL.: "Use of monoclonal antibodies to study in vivo and in vitro activated lymphocytes." TRANSPLANTATION PROCEEDINGS, vol. 21, no. 1 part 1, February 1989 (1989-02), pages 349-350, XP008017039 New York, NY, USA**
- **G. AVERSA ET AL.: "A monoclonal antibody (A6) recognizing a unique epitope restricted to CD45RO and -RB isoforms of the leukocyte common antigen family identifies functional T cell subsets." CELLULAR IMMUNOLOGY, vol. 158, no. 2, 15 October 1994 (1994-10-15), pages 314-328, XP002228858 cited in the application**
- **F. KOLBINGER ET AL.: "Humanization of a mouse anti-human IgE antibody: a potential therapeutic for IgE-mediated allergies." PROTEIN ENGINEERING, vol. 6, no. 8, November 1993 (1993-11), pages 971-980, XP000983997 Oxford, GB cited in the application**
- **P. TEMPEST ET AL.: "A humanized anti-tumor necrosis factor-alpha monoclonal antibody that acts as a partial, competitive antagonist of the template antibody.", HYBRIDOMA, vol. 13, no. 3, 1994, pages 183-190, XP008069399, New York, NY, USA**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 421 191 B1

## Description

Field of the Invention

The present invention relates to humanized antibodies against CD45 antigen isoforms, such as for example monoclonal antibodies (mAbs).

Background of the Invention

One approach in the treatment of a variety of diseases is to achieve the elimination or the inactivation of pathogenic leukocytes and the potential for induction of tolerance to inactivate pathological immune responses.

Organ, cell and tissue transplant rejection and the various autoimmune diseases are thought to be primarily the result of T-cell mediated immune response triggered by helper T-cells which are capable of recognizing specific antigens which are captured, processed and presented to the helper T cells by antigen presenting cell (APC) such as macrophages and dendritic cells, in the form of an antigen-MHC complex, i. e. the helper T-cell when recognizing specific antigens is stimulated to produce cytokines such as IL-2 and to express or upregulate some cytokine receptors and other activation molecules and to proliferate. Some of these activated helper T-cells may act directly or indirectly, i. e. assisting effector cytotoxic T-cells or B cells, to destroy cells or tissues expressing the selected antigen. After the termination of the immune response some of the mature clonally selected cells remain as memory helper and memory cytotoxic T-cells, which circulate in the body and rapidly recognize the antigen if appearing again. If the antigen triggering this response is an innocuous environmental antigen the result is allergy, if the antigen is not a foreign antigen, but a self antigen, it can result is autoimmune disease; if the antigen is an antigen from a transplanted organ, the result can be graft rejection.

The immune system has developed to recognize self from non-self. This property enables an organism to survive in an environment exposed to the daily challenges of pathogens. This specificity for non-self and tolerance towards self arises during the development of the T cell repertoire in the thymus through processes of positive and negative selection, which also comprise the recognition and elimination of autoreactive T cells. This type of tolerance is referred to as central tolerance. However, some of these autoreactive cells escape this selective mechanism and pose a potential hazard for the development of autoimmune diseases. To control the autoreactive T cells that have escaped to the periphery, the immune system has peripheral reg ulatory mechanisms that provide protection against autoimmunity. These mechanisms are a basis for peripheral tolerance.

Cell surface antigens recognized by specific mAbs are generally designated by a CD (Cluster of Differentiation) number assigned by successive International Leukocyte Typing workshops and the term CD45 applied herein refers to the cell surface leukocyte common antigen CD45; and an mAb to that antigen is designated herein as "anti-CD45".

The leukocyte common antigen (LCA) or CD45 is the major component of anti-lymphocyte globulin (ALG). CD45 belongs to the family of transmembrane tyrosine phosphatases and is both a positive and negative regulator of cell activation, depending upon receptor interaction. The phosphatase activity of CD45 appears to be required for activation of Src-family kinases associated with antigen receptor of B and T lymphocytes (Trowbridge IS et al, Annu Rev Immunol. 1994; 12:85-116). Thus, in T cell activation, CD45 is essential for signal 1 and CD45-deficicient cells have profound defects in TCR-mediated activation events.

The CD45 antigen exists in different isoforms comprising a family of transmembrane glycoproteins. Distinct isoforms of CD45 differ in their extracellular domain structure which arise from alternative splicing of 3 variable exons coding for part of the CD45 extracellular region (Streuli MF. et al, J. Exp. Med. 1987; 166:1548-1566). The various isoforms of CD45 have different extra-cellular domains, but have the same transmembrane and cytoplasmic segments having two homologous, highly conserved phosphatase domains of approximately 300 residues. Different isoform combinations are differentially expressed on subpopulations of T and B lymphocytes (Thomas ML. et al, Immunol. Today 1988; 9:320-325). Some monoclonal antibodies recognize an epitope common to all the different isoforms, while other mAbs have a restricted (CD45R) specificity, dependent on which of the alternatively spliced exons (A, B or C) they recognize. For example, monoclonal antibodies recognizing the product of exon A are consequently designated CD45RA, those recognizing the various isoforms containing exon B have been designated CD45RB (Beverley PCL et al, Immunol. Supp. 1988; I:3-5). Antibodies such as UCHL1 selectively bind to the 180 kDa isoform CD45RO (without any of the variable exons A, B or C) which appears to be restricted to a subset of activated T cells, memory cells and cortical thymocytes and is not detected on B cells (Terry LA et al, Immunol. 1988; 64: 331-336).

Aversa G.G et al (1989), Transplantation proceedings, vol.21, No1; pp 349-350, describes the use of three monoclonal antibodies for identifying activation antigens on lymphocytes and to visualize activated T-cells in rejected renal allografts. An antibody termed 'A6' is disclosed.

In WO98/11918 discloses antibodies and uses thereof, to CDR45R. The uses disclosed include the reversal of transplant rejection or treatment of autoimmune disease.

Description of the Figures

Figure 1 shows that the inhibition of primary MLR by the"candidatemAb"is dose-dependent in the range of 0.001 and 10ug/ml."Concentration"is concentration of the"candidate mAb".

Figure 2 shows the plasmid map of the expression vectorHCMV-G1 HuAb-VHQ comprising the heavy chain having the nucleotide sequence SEQ ID NO : 12 (3921-4274) in the complete expression vector nucleotide sequence SEQID NO : 15.

Figure 3 shows the plasmid map of the expression vectorHCMV-G1 HuAb-VHE comprising the heavy chain having the nucleotide sequence SEQ ID NO :11 (3921-4274) in the complete expression vector nucleotide sequence SEQ ID NO : 16.

Figure 4 shows the plasmid map of the expression vector HCMV-KHuAb-humV1 comprising the light chain having the nucleotide sequence SEQ ID NO : 14 (3964-4284) in the complete expression vector nucleotide sequence SEQ ID NO : 17.

Figure 5 shows the plasmid map of the expression vector HCMV-K HuAb-humV2 comprising the light chain having the nucleotide sequence SEQ ID NO : 13 (3926-4246) in the complete expression vector nucleotide sequence SEQID NO : 18.

Description of the Invention

We have now found a humanized antibody which comprises a polypeptide sequence which binds to CD45RO and CD45RB, hereinafter also designated as a "CD45RO/RB humanized antibody ". This humanized antibody according to the invention may induce immunosuppression, inhibit primary T cell responses and induce T cell tolerance. Furthermore, the humanized antibody of the invention inhibit primary mixed lymphocyte responses (MLR). Cells derived from cultures treated withCD45RO/RB humanized antibody preferredly also have impaired proliferative responses in secondary MLR even in the absence ofCD45RO/RB humanized antibody in the secondary MLR. Such impaired proliferative responses in secondary MLR are an indication of the ability of humanized antibody of the invention to induce tolerance. Additionally, in vivo administration ofCD45RO/RB binding molecule to severe combined immunodeficiency (SCID) mice undergoing xeno GVHD following injection with human PBMC may prolong mice survival, compared to control treated mice, even though circulating human T cells may still be detected in CD45RO/RB binding molecule treated mice.

The humanized antibody of the invention has a binding specificity for bothCD45RO and CD45RB.

Preferably the humanized antibody binds to CD45RO isoforms with a dissociation constant (Kd) < 20nM, preferably with a Kd < 15nM or < 10nM, more preferably with a Kd < 5nM. Preferably the humanized antibody binds to CD45RB isoforms with aKd < 50nM, preferably with a Kd < 15nM or < 1 OnM, more preferably with a Kd < 5nM.

In a further preferred embodiment the humanized antibody binds those CD45 isoforms which

1) include the A and B epitopes but not the C epitope of the CD45 molecule ; and/or
2) include the B epitope but not the A and not the C epitope of the CD45 molecule ; and/or
3) do not include any of the A, B or C epitopes of the CD45 molecule.

In yet a further preferred embodiment the humanized antibody does not bind CD45 isoforms which include

1) all of the A, B and C epitopes of the CD45 molecule ; and/or
2) both the B and C epitopes but not the A epitope of the CD45 molecule.

In further preferred embodiments the humanized antibody further

1) recognises memory and in vivo alloactivated T cells ; and/or
2) binds to its target on human T cells, such as for example PEER cells ; wherein said binding preferably is with a Kd < 15nM, more preferably with aKd < 10nM, most preferably with aKd < 5nM ; and/or
3) inhibits in vitroalloreactive T cell function, preferably with anIC50 of about5nM, more preferably with anIC50 of about1 nM, most preferably with anIC50 of about 0,5nM or even0, 1nM ; and/or
4) induces alloantigen-specific T cell tolerance in vitro;and/or
5) prevents lethal xenogeneic graft versus host disease(GvHD) induced in SCID mice by injection of human PBMC when administered in an effective amount.

In a further preferred embodiment the humanized antibody of the invention binds to the same epitope as the monoclonal antibody"A6"as described by Aversa et al., Cellular Immunology 158,314-328 (1994).

Due to the above-described binding properties and biological activities, such humanized antibody of the invention are particularly useful in medicine, for therapy and/or prophylaxis. Diseases in which humanized antibody of the invention are particularly useful include autoimmune diseases, transplant rejection, psoriasis, inflammatory bowel disease and allergies, as will be further set out below.

We have found that a molecule comprising a polypeptide of SEQID NO: 1 and a polypeptide of SEQ ID NO: 2 is a CD45RO/RB binding molecule. We also have found the hypervariable regions CDR1', CDR2'and CDR3' in a CD45RO/RB binding molecule of SEQ ID NO :1, CDR1'having the amino acid sequence Arg-Ala-Ser-Gin-Asn-lie-Gly-Thr-Ser-Ile-Gln (RASQNIGTSIQ), CDR2'having the amino acid sequence er-Ser-Ser-Glu-Ser-Ile-Ser (SSSESIS) and CDR3'having the amino acid sequence Gln-Gln-Ser-Asn-Thr-Trp-Pro-Phe-Thr(QQSNTWPFT).

We also have found the hypervariable regions CDR1, CDR2 and CDR3 in aCD45RO/RB binding molecule of SEQID NO : 2, CDR1 having the amino acid sequence Asn-Tyr-Ile-Ile-His (NYIIH), CDR2 having the amino acid sequence Tyr-Phe-Asn-Pro-Tyr-Asn-His-Gly-Thr Lys-Tyr-Asn-Glu-Lys-Phe-Lys-Gly (YFNPYNHGTKYNEKFKG) and CDR3 having the amino acid sequence Ser-Gly-Pro-Tyr-Ala-Trp-Phe-Asp-Thr (SGPYAWFDT).

CDRs are 3 specific complementary determining regions which are also called hypervariable regions which essentially determine the antigen binding characteristics. These CDRs are part of the variable region, e. g. of SEQ ID NO: 1 or SEQ ID NO: 2, respectively, wherein these CDRs alternate with framework regions (FR's) e. g. constant regions. A SEQ ID NO: 1 is part of a light chain, e. g. of SEQID NO: 3, and a SEQ ID NO : 2 is part of a heavy chain, e. g. of SEQID NO: 4, in a chimeric antibody. The CDRs of a heavy chain together with the CDRs of an associated light chain essentially constitute the antigen binding site of a molecule of the present invention. It is known that the contribution made by a light chain variable region to the energetics of binding is small compared to that made by the associated heavy chain variable region and that isolated heavy chain variable regions have an antigen binding activity on their own. Such molecules are commonly referred to as single domain antibodies.

In one aspect we provide a molecule comprising at least one antigen binding site, e. g. aCD45RO/RB binding molecule, comprising in sequence the hypervariable regions CDR1, CDR2 and CDR3, said CDR1 having the amino acid sequence Asn-Tyr-Ile-Ile-His(NYIIH), said CDR2 having the amino acid sequence Tyr-Phe-Asn-Pro-Tyr-Asn-His Gly-Thr-Lys-Tyr-Asn-Glu-Lys-Phe-Lys-Gly (YFNPYNHGTKYNEKFKG) and said CDR3 having the amino acid sequence Ser-Gly-Pro-Tyr-Ala-Trp-Phe-Asp-Thr (SGPYAWFDT); e. g. and direct equivalents thereof.

In another aspect we provide a molecule comprising at least one antigen binding site, e. g. a CD45RO/RB binding molecule, comprising

a) a first domain comprising in sequence the hypervariable regions CDR1, CDR2 and CDR3, said CDR1 having the amino acid sequence Asn-Tyr-Ile-Ile-His (NYIIH), said CDR2 having the amino acid sequence Tyr-Phe-Asn-Pro-Tyr-Asn-His-Gly-Thr-lys-Tyr-Asn-Glu-Lys-Phe-Lys-Gly (YFNPYNHGTKYNEKFKG) and said CDR3 having the amino acid sequence Ser-Gly-Pro-Tyr-Ala-Trp-Phe-Asp-Thr (SGPYAWFDT);

and b) a second domain comprising in sequence the hypervariable regionsCDR1', CDR2' and CDR3', CDR1' having the amino acid sequence Arg-Ala-Ser-Gln-Asn-Ile-Gly-Thr-Ser-Ile-Gln (RASQNIGTSIQ), CDR2' having the amino acid sequence Ser-Ser Ser-Glu-Ser-Ile-Ser (SSSESIS) and CDR3' having the amino acid sequence Gln-Gln-Ser-Asn-Thr-Trp-Pro-Phe-Thr (QQSNTWPFT), e. g. and direct equivalents thereof.

In a preferred embodiment the first domain comprising in sequence the hypervariable regions CDR1, CDR2 and CDR3 is an immunoglobulin heavy chain, and the second domain comprising in sequence the hypervariable regionsCDR1', CDR2' and CDR3' is an immunoglobulin light chain.

In another aspect we provide a molecule, e. g. a CD45RO/RB binding molecule, comprising a polypeptide of SEQ ID NO: 1 and/or a polypeptide of SEQ ID NO: 2, preferably comprising in one domain a polypeptide of SEQ ID NO: 1 and in another domain a polypeptide of SEQ ID NO: 2, e.g. a chimeric monoclonal antibody, and in another aspect a molecule, e. g. a CD45RO/RB binding molecule, comprising a polypeptide of SEQID NO:3 and/or a polypeptide of SEQ ID NO: 4, preferably comprising in one domain a polypeptide of SEQ.ID NO: 3 and in another domain a polypeptide of SEQ ID NO: 4, e. g. a chimeric monoclonal antibody.

When the antigen binding site comprises both the first and second domains or a polypeptide of SEQID NO:1 or SEQ ID NO : 3, respectively, and a polypeptide of SEQ ID NO: 2 or of SEQ ID NO : 4, respectively, these may be located on the same polypeptide, or, preferably each domain may be on a different chain, e. g. the first domain being part of an heavy chain, e. g. immunoglobulin heavy chain, or fragment thereof and the second domain being part of a light chain, e. g. an immunoglobulin light chain or fragment thereof.

We have further found that a CD45RO/RB humanized antibody according to the present invention is a CD45RO/RB binding molecule in mammalian, e. g. human, body environment. ACD45RO/RB humanized antibody according to the present invention can thus be designated as a monoclonal antibody (mAb), wherein the binding activity is determined mainly by the CDR regions as described above, e. g. said CDR regions being associated with other molecules without binding specificity, such as framework, e.g. constant regions, which are substantially of human origin.

In another aspect we provide a CD45RO/RB binding molecule which is not the monoclonal antibody "A6"as described by Aversa etal., Cellular Immunology 158, 314-328 (1994), which is incorporated by reference for the passages characterizing A6.

In another aspect the present invention provides a CD45RO/RB binding molecule according to the present invention which is a humanised monoclonal antibody.

Examples of aCD45RO/RB binding molecules include chimeric or humanised antibodies e. g. derived from antibodies as produced by B-cells or hybridomas and or any fragment thereof, e. g. F (ab') 2 and Fab fragments, as well as single chain or single domain antibodies. A single chain antibody consists of the variable regions of antibody heavy and light chains covalently bound by a peptide linker, usually consisting of from 10 to 30 amino acids, preferably from 15 to 25 amino acids. Therefore, such a structure does not include the constant part of the heavy and light chains and it is believed that the small peptide spacer should be less antigenic than a whole constant part. By a chimeric antibody is meant an antibody in which the constant regions of heavy and light chains or both are of human origin while the variable domains of both heavy and light chains are of non-human (e. g. murine) origin. By a humanised antibody is meant an antibody in which the hypervariable regions(CDRs) are of non-human (e. g. murine) origin while all or substantially all the other part, e. g. the constant regions and the highly conserved parts of the variable regions are of human origins. A humanised antibody may however retain a few amino acids of the murine sequence in the parts of the variable regions adjacent to the hypervariable regions.

Hypervariable regions, i.e. CDR's may be associated with any kind of framework regions, e. g. constant parts of the light and heavy chains, of human origin. Suitable framework regions are e. g. described in "Sequences of proteins of immunological interest", Kabat, E. A. et al, US department of health and human services, Public health service, National Institute of health. Preferably the constant part of a human heavy chain may be of the IgG1 type, including subtypes, preferably the constant part of a human light chain may be of the κ or λ type, more preferably of the κ type. A preferred constant part of a heavy chain is a polypeptide of SEQ ID NO: 4, (without the CDR1', CDR2' and CDR3' sequence parts which are specified above) and a preferred constant part of a light chain is a polypeptide of SEQID NO: 3 (without the CDR1, CDR2 and CDR3 sequence parts which are specified above).

We also have found a humanised CD45RO/RB antibody comprising a light chain variable region of amino acid SEQ ID NO :7 or of amino acid SEQID NO :8, which comprises CDR1', CDR2'and
CDR3' according to the present invention and a heavy chain variable region of SEQ: ID NO : 9 or of SEQ: ID NO : 10, which comprises CDR1, CDR2 and CDR3 according to the present invention.

In another aspect the present invention provides a humanised CD45RO/RB antibody comprising a heavy chain variable region of SEQ ID NO : 9 or of SEQID NO :10 and a light chain variable region of SEQ ID NO : 7 or of SEQ ID NO : 8.

In another aspect the present invention provides a humanised CD45RO/RB antibody comprising

- a heavy chain variable region of SEQ ID NO : 9 and a light chain variable region of SEQ ID NO: 7,
- a heavy chain variable region of SEQ ID NO : 9 and a light chain variable region of SEQ ID NO : 8,
- a heavy chain variable region of SEQID NO : 10 and a light chain variable region of SEQ ID NO : 7, or
- a heavy chain variable region of SEQ ID NO :10 and a light chain variable region of SEQ ID NO : 8.

"Polypeptide", if not otherwise specified herein, includes any peptide or protein comprising amino acids joined to each other by peptide bonds, having an amino acid sequence starting at the N-terminal extremity and ending at the C-terminal extremity. Preferably the polypeptide of the present invention is a monoclonal antibody, more preferred is a chimeric (V-grafted) or humanised(CDR-grafted) monoclonal antibody.-The humanised (CDRgrafted) monoclonal antibody may or may not include further mutations introduced into the framework (FR) sequences of the acceptor antibody.

A functional derivative of a polypeptide as used herein includes a molecule having a qualitative biological activity in common with a polypeptide to the present invention, i. e. having the ability to bind toCD45RO and CD45RB. A functional derivative includes fragments and peptide analogs of a polypeptide according to the present invention.
Fragments comprise regions within the sequence of a polypeptide according to the present invention, e. g. of a specified sequence. The term "derivative" is used to define amino acid sequence variants, and covalent modifications of a polypeptide according to the present invention. e. g. of a specified sequence. The functional derivatives of a polypeptide e.g. of a specified sequence, preferably have at least about 65%, more preferably at least about 75%, even more preferably at least about 85%, most preferably at least about 95% overall sequence homology with the amino acid sequence of a polypeptide according to the present invention, e. g. of a specified sequence, and substantially retain the ability to bind to CD45RO and CD45RB.

The term "covalent modification" includes modifications of a polypeptide according to the present invention, e. g. of a specified sequence; or a fragment thereof with an organic proteinaceous or non-proteinaceous derivatizing agent, fusions to heterologous polypeptide sequences, and post-translational modifications. Covalent modified polypeptides, e. g. of a specified sequence, still have the ability bind toCD45RO and CD45RB by crosslinking Covalent modifications are traditionally introduced by reacting targeted amino acid residues with an organic derivatizing agent that is capable of reacting with selected sides or terminal residues, or by harnessing mechanisms of post-translational modifications that function in selected recombinant host cells. Certain post-translational modifications are the result of the action of recombinant host cells on the expressed polypeptide. Glutaminyl and asparaginyl residues are frequently post-translationally deamidated to the corresponding glutamyl and aspartyl residues. Alternatively, these residues are deaminated under mildly acidic conditions. Other post-translational modifications include hydroxylation of proline and lysine, phosphorylation of hydroxyl groups of seryl, tyrosine or threonyl residues, methylation of the $\alpha$-amino groups of lysine, arginine, and histidine side chains, see e. g. T. E. Creighton, Proteins: Structure and Molecular Properties, W. H. Freeman & Co., San Francisco, pp. 7986 (1983). Covalent modifications e. g. include fusion proteins comprising a polypeptide according to the present invention, e. g. of a specified sequence and their amino acid sequence variants, such as immunoadhesins, and N-terminal fusions to heterologous signal sequences.

"Homology" with respect to a native polypeptide and its functional derivative is defined herein as the percentage of amino acid residues in the candidate sequence that are identical with the residues of a corresponding native polypeptide, after aligning the sequences and introducing gaps, if necessary, to achieve the maximum percent homology, and not considering any conservative substitutions as part of the sequence identity. Neither N-or C-terminal extensions nor insertions shall be construed as reducing identity or homology.

Methods and computer programs for the alignment are well known.

"Amino acid (s)" refer to all naturally occurring L-$\alpha$-amino acids, e. g. and including D-amino acids. The amino acids are identified by either the well known single-letter or three-letter designations.

The term "amino acid sequence variant" refers to molecules with some differences in their amino acid sequences as compared to a polypeptide according to the present invention, e. g. of a specified sequence. Amino acid sequence variants of a polypeptide according to the present invention, e. g. of a specified sequence, still have the ability to bind toCD45RO and CD45RB. Substitutional variants are those that have at least one amino acid residue removed and a different amino acid inserted in its place at the same position in a polypeptide according to the present invention, e. g. of a specified sequence. These substitutions may be single, where only one amino acid in the molecule has been substituted, or they may be multiple, where two or more amino acids have been substituted in the same molecule.
Insertional variants are those with one or more amino acids inserted immediately adjacent to an amino acid at a particular position in a polypeptide according to the present invention, e. g. of a specified sequence. Immediately adjacent to an amino acid means connected to either the $\alpha$-carboxy or a-amino functional group of the amino acid. Deletional variants are those with one or more amino acids in a polypeptide according to the present invention, e. g. of a specified sequence, removed. Ordinarily, deletional variants will have one or two amino acids deleted in a particular region of the molecule.

We also have found the polynucleotide sequences of

- GGCCAGTCAGAACATTGGCACAAGCATACAGTG, encoding the amino acid sequence of CDR1,
- TTCTTCTGAGTCTATCTCTGG ; encoding the amino acid sequence of CDR 2,
- ACAAAGTAATACCTGGCCATTCACGTT encoding the amino acid sequence of CDR 3,
- TTATATTATCCACTG, encoding the amino acid sequence of CDR1',
- TTTTAATCCTTACAATCATGGTACTAAGTACAATGAGAAGTTCAAAGGCAG encoding the amino acid sequence of CDR2',
- AGGACCCTATGCCTGGTTTGACACCTG encoding the amino acid sequence of CDR3',
- SEQ ID NO : 5 encoding a polypeptide of SEQ ID NO: 1, i.e. the variable region of a light chain of an mAb;
- SEQ ID NO : 6 encoding a polypeptide of SEQ ID NO : 2, i.e. the variable region of the heavy chain of an mAb;
- SEQ ID NO :11 encoding a polypeptide of SEQ ID NO :9. i.e. a heavy chain variable region including CDR1, CDR2 and CDR3 according to the present invention;
- SEQ ID NO : 12 encoding a polypeptide of SEQ ID NO : 10, i.e. a heavy chain variable region including CDR1, CDR2 and CDR3 according to the present invention;
- SEQ ID NO : 13 encoding a polypeptide of SEQ ID NO : 7, i. e. a light chain variable region including CDR1', CDR2'and CDR3'according to the present invention; and
- SEQ ID NO : 14 encoding a polypeptide of SEQID NO : 8, i. e. a light chain variable region including CDR1', CDR2'and CDR3'according to the present invention.

In another aspect the present invention provides isolated polynucleotides encoding a polypeptide of SEQ ID NO : 7 or SEQ.ID NO: 8 and a polypeptide of SEQ ID NO : 9 or SEQ ID NO : 10; e. g. encoding

- a polypeptide of SEQ ID NO : 7 and a polypeptide of SEQ ID NO : 9,
- a polypeptide of SEQ ID NO : 7 and a polypeptide of SEQ ID NO : 10,
- a polypeptide of SEQ ID NO : 8 and a polypeptide of SEQ ID NO : 9, or
- a polypeptide of SEQID NO : 8 and a polypeptide of SEQID NO : 10.

"Polynucleotide", if not otherwise specified herein, includes any polyribonucleotide or polydeoxyribonucleotide, which may be unmodified RNA or DNA, or modified RNA or DNA, including without limitation single and double stranded RNA, and RNA that is a mixture of single-and double-stranded regions.

A polynucleotide according to the present invention, e.g. a polynucleotide encoding the amino acid sequence CDR1, CDR2, CDR3, CDR1', CDR2', CDR3', or of SEQ.ID NO :1, SEQ.ID NO : 2, SEQ.ID NO : 3, SEQ.ID NO : 4, SEQ ID NO : 7, SEQ ID NO : 8, SEQ ID NO : 9 or SEQ ID NO : 10, respectively, such as a polynucleotide of SEQ ID NO : 5, SEQ ID NO : 6, SEQ ID NO :11, SEQ ID NO : 12, SEQ ID NO : 13 or SEQ ID NO: 14, respectively, includes allelic variants thereof and/or their complements; e. g. including a polynucleotide that hybridizes to the nucleotide sequence of SEQ ID NO: 5, SEQ ID NO : 6, SEQID NO :11, SEQ ID NO : 12, SEQ ID NO : 13 or SEQ ID NO : 14, respectively; e. g. encoding a polypeptide having at least 80% identity to SEQ IDN0 :1, SEQ IDN0 : 2, SEQ ID NO : 3, SEQID NO : 4, SEQ ID NO : 7, SEQ ID NO : 8, SEQ ID NO : 9 or SEQ ID NO : 10, respectively, e. g. including a functional derivative of said polypeptide, e. g. said functional derivative having at least 65% homology with SEQ ID NO :1, SEQ ID NO : 2, SEQ ID NO :3, SEQ ID NO : 4, SEQ ID NO : 7, SEQ ID NO : 8, SEQ ID NO : 9 or SEQ ID NO : 10, respectively, e. g. said functional derivative including covalent modifications of SEQID NO : 1, SEQ ID NO : 2, SEQ ID NO : 3, SEQ ID NO : 4, SEQ ID.NO : 7, SEQID NO : 8, SEQID NO : 9 or SEQID NO : 10, respectively, e. g. said functional derivative including amino acid sequence variants of SEQID NO :1, SEQID NO : 2, SEQ ID.NO : 3, SEQ ID NO : 4, SEQ ID NO : 7, SEQ ID NO : 8, SEQ ID NO : 9 or SEQ ID NO : 10, respectively ; e. g. a SEQ ID NO : 5, SEQ ID NO : 6, SEQID NO : 11, SEQ ID NO : 12, SEQ ID.NO : 13 or SEQ ID NO : 14, respectively includes a sequence, which as a result of the redundancy (degeneracy) of the genetic code, also encodes a polypeptide of SEQID NO :1, SEQ ID NO : 2, SEQ ID NO : 3, SEQ ID NO : 4, SEQ ID NO :7, SEQ ID NO : 8, SEQ ID NO: 9 or SEQ.ID NO : 10, respectively, or encodes a polypeptide with an amino acid sequence which has at least 80% identity with the amino acid sequence of SEQ ID NO : 1, SEQID NO : 2, SEQ.ID NO :3, SEQ ID NO : 4, SEQID NO : 7, SEQ ID NO : 8, SEQ ID NO : 9 or SEQ ID NO : 10, respectively.

A CD45RO/RB humanized antibody may be produced by recombinant DNA techniques. Thus, one or more DNA molecules encoding theCD45RO/RB may be constructed, placed under appropriate control sequences and transferred into a suitable host (organism) for expression by an appropriate vector.

In another aspect the present invention provides a polynucleotide which encodes a single, heavy and/or a light chain of aCD45RO/RB humanized antibody according to the present invention; and the use of a polynucleotide according to the present invention for the production of a CD45RO/RB humanized antibody according to the present invention by recombinant means.

A CD45RO/RB humanized antibody may be obtained according, e. g. analogously, to a method as conventional together with the information provided herein, e. g. with the knowledge of the amino acid sequence of the hypervariable or variable regions and the polynucleotide sequences encoding these regions. A method for constructing a variable domain gene is e. g. described in EP 239 400 and may be briefly summarized as follows : A gene encoding a variable region of a mAb of whatever specificity may be cloned. The DNA segments encoding the framework and hypervariable regions are determined and the DNA segments encoding the hypervariable regions are removed. Double stranded synthetic CDR cassettes are prepared by DNA synthesis according to the CDR and CDR' sequences as specified herein. These cassettes are provided with sticky ends so that they can be! gated at junctions of a desired framework of human origin. Polynucleotides encoding single chain antibodies may also be prepared according to, e.g. analogously, to a method as conventional. A polynucleotide according to the present invention thus prepared may be conveniently transferred into an appropriate expression vector.

Appropriate cell lines may be found according, e. g. analogously, to a method as conventional. Expression vectors, e. g. comprising suitable promotor (s) and genes encoding heavy and light chain constant parts are known e. g. and are commercially available.

Appropriate hosts are known or may be found according, e. g. analogously, to a method as conventional and include cell culture or transgenic animals.

In another aspect the present invention provides an expression vector comprising a polynucleotide encoding a CD45RO/RB humanized antibody according to the present invention.

In another aspect the present invention provides

- An expression system comprising a polynucleotide according to the present invention wherein said expression system or part thereof is capable of producing a CD45RO/RB humanized antibody according to the present invention, when said expression system is present in a compatible host cell ;
  and
- An isolated host cell which comprises an expression system as defined above.

We have further found that a CD45RO/RB humanized antibody according to the present invention inhibit primary alloimmune responses in a dose-dependent fashion as determined by in vitro MLR. The results indicate that the cells which had been alloactivated in the presence of a CD45RO/RB humanized antibody according to the present invention are impaired in their responses to alloantigen. This confirms the indication that a CD45RO/RB humanized antibody according to the present invention can act directly on the effector alloreactive T cells and modulate their function. In addition, the functional properties of T cells derived from the primary MLR were further studied in restimulation experiments in secondary MLR, using specific stimulator cells or third-party simulators to assess the specificity of the observed functional effects. We have found that the cells derived from primary MLRs in which a CD45RO/RB humanized antibody according to the present invention is present, were impaired in their ability to respond to subsequent optimal stimulation with specific stimulator cells, although there was no antibody added to the secondary cultures. The specificity of the inhibition was demonstrated by the ability of cells treated with a CD45R0/RB humanized antibody according to the present invention to respond normally to stimulator cells from unrelated third-party donors. Restimulation experiments using T cells derived from primary MLR cultures thus indicate that the cells which had been alloactivated a CD45RO/RB according to the present invention are hyporesponsive, i. e. tolerant, to the original alloantigen. Further biological activities are described in example 7.

Furthermore we have found that cell proliferation in cells pre-treated with aCD45RO/RB humanized antibody according to the present invention could be rescued by exogenousIL-2. This indicates that treatment of alloreactive T cells with a CD45RO/RB humanized antibody, according to the present invention induces a state of tolerance. Indeed, the reduced proliferative responses observed in cells treated with a CD45RO/RB humanized antibody according to the present invention, was due to impairment of T cell function, and these cells were able to respond to exogenous IL-2, indicating that these cells are in an anergic, true unresponsive state. The specificity of this response was shown by the ability of cells treated with a CD45RO/RB humanized antibody according to the present invention to proliferate normally to unrelated donor cells to the level of the control treated cells.

In addition experiments indicate that the binding of a CD45RO/RB humanized antibody according to the present invention to CD45RO and CD45RB may inhibit the memory responses of peripheral blood mononuclear cells (PBMC) from immunized donors to specific recall antigen. Binding of a CD45RO/RB humanized antibody according to the present invention to CD45RO and CD45RB thus is also effective in inhibiting memory responses to soluble Ag. The ability of a CD45RO/RB humanized antibody according to the present invention to inhibit recall responses to tetanus in PBMC from immunized donors indicate that the CD45RO/RB humanized antibody according to the present invention is able to target and modulate the activation of memory T cells. E. g. these data indicate that a CD45RO/RB humanized antibody according to the present invention in addition to recognizing alloreactive and activated T cells is able to modulate their function, resulting in induction of T cell anergy. This property may be important in treatment of ongoing immune responses to autoantigens and allergens and possibly to alloantigens as seen in autoimmune diseases, allergy and chronic rejection, and diseases, such as psoriasis, inflammatory bowel disease, where memory responses play a role in the maintenance of disease state. It is believed to be an important feature in a disease situation, such as in autoimmune diseases in which memory responses to autoantigens may play a major role for the disease maintenance.

We have also found that a CD45RO/RB humanized antibody according to the present invention may modulate T cell proliferative responses in a mixed lymphocyte response (MLR) in vivo, i.e. a CD45RO/RB binding molecule according to the present invention was found to have corresponding inhibitory properties in vivo testing.

A CD45RO/RB humanized antibody according to the present invention may thus have immunosuppressive and tolerogenic properties and may be useful for in vivo and ex-vivo tolerance induction to alloantigens, autoantigens, allergens and bacterial flora antigens, e. g. a CD45RO/RB binding molecule according to the present invention may be useful in the treatment and prophylaxis of diseases e. g. including autoimmune diseases, such as, but not limited to, rheumatoid arthritis, autoimmune thyroditis, Graves disease, type I and type 11 diabetes, multiple sclerosis, systemic lupuserythematosus, Sjogren syndrome,scieroderma, autoimmunegastritis, glomerulonephritis, transplant rejection, e. g. organ and tissue allograft and xenograft rejection, graft versus host disease (GVHD), and also psoriasis, inflammatory bowel disease and allergies.

In another aspect the present invention provides the use of a CD45RO/RB humanized antibody according to the present invention as a pharmaceutical, e. g. in the treatment and prophylaxis of autoimmune diseases, transplant rejection, psoriasis, inflammatory bowel disease and allergies.

In another aspect the present invention provides a CD45RO/RB humanized antibody according to the present invention for the production of a medicament in the treatment and prophylaxis of diseases associated with autoimmune diseases, transplant rejection, psoriasis, inflammatory bowel disease and allergies.

In another aspect the present invention provides a pharmaceutical composition comprising a CD45RO/RB humanized antibody according to the present invention in association with at least one pharmaceutical acceptable carrier or diluent.

A pharmaceutical composition may comprise further, e. g. active, ingredients, e. g. other immunomodulatory antibodies such as, but not confined to anti-ICOS, anti-CD154, antCD134L or recombinant proteins such as, but not confined to rCTLA-4(CD152), rOX40(CD134), or immunomodulatory com pounds such as, but not confined to cyclosporin A, FTY720, RAD, rapamycin, FK506,15-deoxyspergualin, steroids.

In another aspect we provide a method of treatment and/or prophylaxis of diseases associated with autoimmune diseases, transplant rejection, psoriasis, inflammatory bowel disease and allergies comprising administering to a subject in need of such treatment and/or prophylaxis an effective amount of a CD45RO/RB binding molecule according to the present invention, e. g. in the form of a pharmaceutical composition according to the present invention.

Autoimune diseases to be treated with of the present invention further include, but are not limited to, rheumatoid arthritis, autoimmune thyroditis, Graves disease, type I and type 11 diabetes, multiple sclerosis, systemic lupus erythematosus, Sjogren syndrome, scleroderma, autoimmune gastritis, glomerulonephritis ; transplant rejection, e. g. organ and tissue allograft and xenograft rejection and graft-versus-host disease (GVHD).

## EXAMPLES

The invention will be more fully understood by reference to the following examples. They should not, however, be construed as limiting the scope of the invention. In the following examples all temperatures are in degree Celsius.

The "candidate mAb" or "chimeric antibody" is a CD45RO/RB binding molecule according to the present invention comprising light chain of SEQ ID NO:3 and heavy chain of SEQ ID NO:4.

The following abbreviations are used:

| | |
|---|---|
| ELISA | enzyme linked immuno-sorbant assay |
| FACS | fluorescence activated cell sorting |
| FITC | fluorescein isothiocyanate |
| FBS | foetal bovine serum |
| GVHD | graft-vs-host disease |
| HCMV | human cytomegalovirus promoter |
| IgE | immunoglobulin isotype E |
| IgG | immunoglobulin isotype G |
| PBS | phosphate-buffered saline |
| PCR | polymerase chain reaction |
| xGVHD | xeno-graft-vs-host disease |

### Example 1: Primary mixed lymphocyte response (MLR)

Cells

Blood samples are obtained from healthy human donors. Peripheral blood mononuclear cells (PBMC) are isolated by centrifugation over Ficoll-Hypaque (Pharmacia LKB) from leukocytes from whole peripheral blood, leukopheresis or buffy coats with known blood type, but unknown HLA type. In some MLR experiments, PBMC are directly used as the stimulator cells after the irradiation at 40 Gy. In the other experiments, T cells were depleted from PBMC by using CD2 or CD3 Dynabeads (Dynal, Oslo, Norway). Beads and contaminating cells are removed by magnetic field. T cell-depleted PB MC are used as simulator cells after the irradiation.
PBMC, $CD3^+$ T cells or $CD4^+$ T cells are used as the responder cells in MLR. Cells are prepared from different donors to stimulator cells. $CD3^+$ T cells are purified by negative selection using anti-CD16 mAb (Zymed, CA), goat anti-mouse IgG Dynabeads, anti-CD14 Dynabeads, CD19 Dynabeads. In addition anti-CD8 Dynabeads are used to purify CD4 T cells. The cells obtained are analyzed by FACScan or FACSCalibur (Becton Dickinson & Co., CA) and the purity of the cells obtained was >75%. Cells are suspended in RPM11640 medium, supplemented with 10 % heat-inactivated FBS, penicillin, streptomycin and L-glutamine.

*Reagents*

The chimeric anti-CD45RO/RB mAb "candidate mAb" and an isotype matched control chimeric antibody is also generated. Mouse (Human) control $IgG_1$ antibody specific for KLH (keyhole limpet hemocyanin) or recombinant human IL-10 is purchased from BD Pharmingen (San Diego, CA). Anti-human CD154 mAb 5c8 is according to Lederman et al 1992.

*Primary Mixed lymphocyte response (MLR)*

Aliquots of 1 x $10^5$ PBMC or 5 X $10^4$ of $CD3^+$ or $CD4^+$ cells are mixed with 1x$10^5$ irradiated PBMC or 5 x $10^4$ T cells-depleted irradiated (50 Gy) PBMC in the each well of 96-well culture plates (Costar, Cambridge, MA) in the presence of the indicated mAb or absence of Ab. In some experiments, $F(ab')_2$ fragment of goat anti-mouse Ig or goat anti-human Ig specific for Fc portion (Jackson ImmunoResearch, West Grove, PA) is added at 10 μg/ml in addition to the candidate mAb To ensure optimal in vitro cross-linking of the target CD45 molecules. The mixed cells are cultured for 4 or 5 days at 37°C in 5% $CO_2$ and proliferation is determined by pulsing the cells with $^3$H-thymidine for the last 16 - 20 hours of culture.

Other experiments are similar to those described above, but with the following exceptions: 1) Medium used is EX-VIVO (Bio-Whittaker) containing 10% FBS and 1% human plasma; 2) Anti-mouse total IgG (5 μg/ml) is used as secondary cross-linking step; 3) Irradiation of stimulator cells is 60 Gy.

Primary MLR is performed in the presence of the "candidate mAb" or control chimeric $IgG_1$ (10 μg/ml) both with a second step reagent, $F(ab')_2$ fragment of goat anti-human Ig specific for Fc portion (10 μg/ml). Percentage inhibition by the "candidate mAb" is calculated in comparison with the cell proliferation in the presence of control $IgG_1$. Results are shown in TABLE 1 below:

TABLE 1

| Inhibition of primary MLR by 10 μg/ml of a candidate mAb according to the present invention | | |
|---|---|---|
| **Responder** | **Stimulator (Irr. PBMC)** | **% of Inhibition** |
| #211 CD4 | #219 CD3 | 63.51 |
| #220 CD4 | #219 CD3 depl. | 63.07 |
| #227 CD4 | #220 CD3 depl. | 65.96 |
| #229 CD4 | #219 CD3 depl. | 50.76 |
| Average± SD | | 60.83 ±6.83* |
| * Significantly different from control value (P<0.001) | | |

A candidate mAb according to the present invention inhibits primary MLR as can be seen from TABLE 1. The average inhibitory effect is 60.83 ± 6.83 % in four different donors-derived $CD4^+$ T cells and statistically significant.

The inhibition of primary MLR by the "candidate mAb" is shown to be dose-dependent in the range of 0.001 and 10 μg/ml of the "candidate mAb" as shown in Figure 1.

The $IC_{50}$ for the inhibition of primary MLR by a "candidate mAb" is determined from the results of three separate MLR experiments using one donor PBMC as responder cells. Thus, responder $CD4^+$ T cells from Donor #229 and #219 and irradiated PBMC depleted of T cells as stimulators are mixed in the presence of a "candidate mAb" or control chimeric Ab with 10 μg/ml of $F(ab')_2$ fragment of goat anti-human Ig. Experiments are repeated 3 times and percentage of proliferation in the presence of a "candidate mAb" is calculated in comparison with the T cell proliferation in the presence of control Ab. $IC_{50}$ value is determined using Origin (V. 6.0®). The cellular activity $IC_{50}$ value is calculated to be 0.87 ± 0.35 nM (0.13 ± 0.052 μg/ml).

**<u>Example 2:</u> Secondary MLR**

In order to assess whether a "candidate mAb" induces unresponsiveness of $CD4^+$ T cells to specific alloantigens, secondary MLR is performed in the absence of any antibodies after the primary MLC. $CD4^+$ T cells are cultured with irradiated allogeneic stimulator cells (T cells-depleted PBMC) in the presence of the indicated antibody in 96-well culture plates for 10 days (primary MLC). Then, cells are collected, layered on a Ficoll-Hypaque gradient to remove dead cells, washed twice with RPMI, and restimulated with the same stimulator, 3$^{rd}$ party stimulator cells or IL-2 (50 U/ml). The cells are cultured for 3 days and the proliferative response is determined by pulsing the cells with $^3$H-thymidine for the last 16 - 20 hours of culture.

Specifically, $CD4^+$ T cells are cultured with irradiated allogeneic stimulator cells (T cells-depleted PBMC taken from other donors) in the presence of 10 μg/ml of the "candidate mAb", control IgG1 chimeric Ab and $F(ab')_2$fragment of goat anti-human Ig. Primary MLR proliferation is determined on day 5. For secondary MLR, the responder and stimulator cells are cultured for 10 days in the presence of the "candidate mAb", then the cells are harvested, washed twice in RPMI1640 and restimulated with specific stimulator, third-party stimulators or IL-2 (50 U/ml) in the absence of any Ab. Cell proliferation is determined on day 3. Results set out in TABLE 2:

TABLE 2

| Responder CD4+ T cells Donor # | % Inhibition of $2^{ry}$ MLR |
|---|---|
| #211 | 49.90* |
| #220 | 59.33* |
| #227 | 58.68* |
| * Significantly different from control value (p=<0.001 determined by t-test, SigmaStat V.2.03). # p=<0.046 | |

In order to test whether the impaired proliferation is due to unresponsivess as a consequence of the treatment with a "candidate mAb", the cells derived from primary MLR are cultured in the presence of IL-2 (50 U/ml). Addition of IL-2 results in the rescue of proliferative responses of the T cells which had been treated with a "candidate mAb" in primary MLR, to levels similar to those observed in the presence of $IgG_1$ control Ab. These data indicate that the impaired secondary response in T cells treated with a "candidate mAb" is due to to functional alteration of the responder T cells which become unresponsive to the specific stimulator cells.
Percentage inhibition is calculated according to the following formula:

$$\frac{\text{c.p.m. with control Ab} - \text{c.p.m. with "candidate mAb}}{\text{c.p.m. with control Ab}} \times 100$$

Statistical analysis is performed using SigmaStat (Vers. 2.03).
The data is analyzed by two-way ANOVA followed by Dunnett method. In all test procedures probabilities <0.05 are considered as significant. In some experiments t-test is used (SigmaStat V.2.03).

**<u>Example 3:</u> In vivo survival studies in SCID-mice**

*Engraftment of hu-PBL in SCID mice*

Human peripheral blood mononuclear cells (PBMC) are injected intraperitoneally into SCID mice C.B 17 /GbmsTac-*Prkdc*$^{scid}$ *Lysr*$^{bg}$ mice (Taconic, Germantown, NY) in an amount sufficient to induce a lethal xenogeneic graft-versus-host disease (xGvHD) in >90% of the mice within 4 weeks after cell transfer. Such treated SCID mice are hereinafter designated as hu-PBL-SCID mice

*Mab-treatment of hu-PBL-SCID mice*

Hu-PBL SCID mice are treated with a "candidate mAb" or mouse or chimeric isotype matched mAb controls at day 0, immediately after PBMC injection, at day 3, day 7 and at weekly intervals thereafter. Mabs are delivered subcutaneously in 100 µl PBS at a final concentration of 5 mg/kg body weight The treatment was stopped when all control mice were dead.

*Evaluation of treatment results*

The main criterion to assess the efficacy of a "candidate mAb" in this study was the survival of the hu-PBL-SCID mice. The significance of the results is evaluated by the statistical method of survival analysis using the Log-rank test (Mantel method) with the help of the Systat v9.01 software. The method of survival analysis is a non-parametric test, which not only consider whether a particular mouse is still alive but also whether if it was sacrificed for reasons irrelevant to the treatment/disease such as the requirement of perform in vitro analysis with its organs/cells. Biopsies of liver, lung, kidney and spleen are obtained from dead mice for further evaluation. In addition, hu-PBL-SCID mice are weighed at the beginning (before cell transfer) and throughout (every two days) the experiment as an indirect estimation of their health status. Linear regression lines were generated using the body weight versus days post-PBMC transfer values obtained from each mouse and subsequently, their slopes (control versus anti-CD45 treated mice) were com pared using the non-parametric Mann-Whitney test.

*Results*

All hu-PBL-SCID mice treated with mouse mAb controls had infiltrated human leukocytes in the lung, liver and spleen and died (4/4) within ca. 2 to 3 weeks after cell transfer. Death is a likely consequence of xGvHD. Control mAb-treated mice furthermore lost weight in a linear manner, ca. 10% and more within 3 weeks.
All hu-PBL-SCID mice treated with a "candidate mAb" survived (4/4) without any apparent sign of disease more than 4 weeks, even although "candidate mAb"-treatment was stopped after 3 weeks. "Candidate mAb"-treated mice increased weight in a linear manner, up to ca. 5% within 4 weeks.

**Example 4: Expression of antibodies of the invention**

Expression of humanised antibody comprising a SEQ ID NO:7, SEQ ID NO:8, SEQ ID NO:9, or SEQ ID NO:10

Expression vectors according to the plasmid map shown in Figures 2 to 5 are constructed, comprising the corresponding nucleotides encoding the amino acid sequence of humanised light chain variable region humV1 (SEQ ID NO:7), humanised light chain variable region humV2 (SEQ ID NO:8), humanised heavy chain variable region VHE (SEQ ID NO:9), or humanised heavy chain variable region VHQ (SEQ ID NO:10), respectively. These expression vectors have the DNA (nucleotide) sequences SEQ ID NO 15, SEQ ID NO 16, SEQ ID NO 17, or SEQ ID NO 18, respectively.

Construction of humanised antibody heavy and light chain expression vectors

Human kappa light chain expression vectors for versions VLh and VLm

In order to construct the final expression vector encoding for the complete humanised light chain of human kappa isotype, DNA fragments encoding the complete light chain variable regions (VLh and VLm) were excised from the VLh and VLm containing PCR-Script cloning vectors (Stratagene) (VLm region) using HindIII and BglII. The gel-purified fragments were then subcloned into the HindIII and BamHI sites of C21-HCMV Kappa expression vector which was created during construction of the humanised anti-IgE antibody TESC-21 (Kolbinger et al 1993) and which originally received from M. Bendig (MRC Collaborative Centre, London, UK) (Maeda et al. 1991). The ligation products were purified by phenol/chloroform extraction, and electroporated into electrocoporation-competent Epicurian Coli® XL1-Blue strain (Cat N° #200228, Stratagene). After plating on LB/amp agar plates overnight at 37°C, each 12 colonies were picked to prepare plasmid DNA from a 3 ml culture using the BioRobot 9600 (Qiagen). This yielded the light chain expression vectors for the humanised antibody versions VLh and VLm, respectively, as further described in the Figures.

Human gamma-1 heavy chain expression vectors for VHQ

For the construction of the VHQ expression vector, a step-wise approach was taken. First, the complete variable region of VHQ was assembled by PCR according to the methology as described in Kolbinger et al 1993 (Protein Eng. 1993 Nov; 6(8):971-80) and subcloned into the C21-HCMV-gamma-1 expression from which the C21 insert had been removed using the same enzymes. A HindIII/BamHI fragment of PCRScript clone VHQ containing the complete variable region was then subcloned into expression vector C21-HCMV-gamma-1 cleaved with the same enzymes. This yielded the final expression vector for the humanised antibody version VHQ.

Human gamma-1 heavy chain expression vectors for VHE

The construction of the final VHE expression vector encoding for the complete humanised heavy chain of human gamma-1 isotype was achieved by directly ligating a HindIII and BamHI restricted PCR fragment encoding the variable region into the HindIII and BamHI sites of C21-HCMV gamma-1 expression vector which was created during construction of the humanised anti-IgE antibody TESC-21 (Kolbinger et al 1993) and which was also originally received from M. Bendig (MRC Collaborative Centre, London, UK) (Maeda et al. 1991 ).

Transient expression in COS cells

The following transfection protocol is adapted for adherent COS cells in 150 mm cell culture dishes, using SuperFect™ Transfection Reagent (Cat. N°301305, Qiagen). The four different expression vectors described above are used for transient transfection of cells. For expression of humanised antibody, each of two clones containing heavy chain inserts (VHE or VHQ, respectively) are co-transfected into cells with each of the two clones encoding for the light chains (humV1 or humV2, respectively), in total 4 different combinations of heavy and light chain expression vectors (VHE/humV1, VHE/humV2, VHQ/humV1 and VHQ/humV2). Before transfection, the plasmids are linearized with the restriction endonuclease PvuI which cleaves in the region encoding the resistance gene for ampicillin.
The day before transfection, 4 x $10^6$ COS cells in 30 ml of fresh culture medium are seeded in 150 mm cell culture dishes. Seeding at this cell density generally yielded 80% confluency after 24 hours. On the day of transfection, four different combinations of linearized heavy-and light-chain DNA expression vectors (15 μg each) are diluted in a total volume of 900 μl of fresh medium without serum and antibiotics. 180 μl of SuperFect Transfection Reagent is then mixed thoroughly with the DNA solution. The DNA mixture is incubated for 10 min at room temperature to allow complex formation. While complex formation takes place, the growth medium is removed from COS cell cultures, and cells are washed once with PBS. 9 ml of fresh culture medium (containing 10% FBS and antibiotics) are then added to each reaction tube containing the transfection complexes and well mixed. The final preparation is immediately transferred to each of 4 cultures to be transfected and gently mixed. Cell cultures are then incubated with the DNA complexes for 3 hours at 37°C and 5% CO2. After incubation, the medium containing transfection complexes is removed and replaced with 30 ml of fresh culture medium. At 48 hr post transfection, the culture supernatants are harvested.

Concentration of culture supernatants

For ELISA and FACS analysis, the culture supernatants collected from COS cells transfected with heavy-and light-chain plasmids are concentrated as follows. 10 mi of each supernatant are added to Centriprep YM-50 Centrifugal Filter Devices (Cat. N 4310,
Millipore) as described by the manufacturer. The Centriprep filters are centrifuged for 10 min at 3000 rpm at room temperature. The centrifugation step is then repeated ag ain with the remaining 20 ml of supernatant using only 5 min of centrifugation and supervising the concentration evolution. The intermediate 500 ul of concentrated supernatant is recovered, transferred to new Microcon Centrifugal Filter Devices (Cat. N 42412, Microcon) and further concentrated following the manufacturer's protocol. The concentrated supernatants are centrifuged four times for 24 min at 3000 rpm at room temperature, one time for 10 min at 6000 rpm and then, three times for 5 min, always supervising the concentration evolution.
The final volume of concentrated conditioned medium achieved is 100-120 ul corresponding to a 250 to 300-fold concentration of original culture medium and is stored at 4°C until use. For comparison and control, culture medium from untransfected cells is similarly concentrated, using the same centrifugation protocol described above.

Example 5: Determination of recombinant humanized IgG expression by ELISA

To determine IgG concentrations of recombinant humanized antibody expressed in the culture supernatants, a sandwich ELISA protocol has been developed and optimized using human IgG as standard. Flat bottom 96-wellmicrofiter plates (Cat. N 4-39454, Nunc Immunoplate Maxisorp) are coated overnight at 4°C with100 μl of goat anti-human IgG (whole molecule,Cat. N 11011, SIGMA) at the final concentration of 0.5μg/ml in PBS. Wells are then washed 3 times with washing buffer (PBS containing 0.05% Tween 20) and blocked for 1.5 hours at 37°C with blocking buffer (0.5% BSA in PBS). After 3 washing cycles, the antibody samples and the standard human IgG (Cat. No.14506, SIGMA) are prepared by serial 1.5-fold dilution in blocking buffer. 100 μl of diluted samples or standard are transferred in duplicate to the coated plate and incubated for 1 hour at room temperature. After incubation, the plates are washed 3 times with washing buffer and subsequently incubated for 1 hour with100 μl of horseradish peroxidase-conjugated goat anti-human IgG kappa-light chain (Cat.N° A-7164, SIGMA) diluted at 1/4000 in blocking buffer. Control wells received 100pi of blocking buffer or concentrated normal culture medium. After washing, the colorimetric quantification of bound peroxidase in the sample and standard wells is performed, using a TMB Peroxidase EIA Substrate Kit (Cat. N 172-1067, Bio-Rad) according to the manufacturer's instructions.
The peroxidase mixture is added at 100 μl per well and incubated for 30 min at room temperature in the dark. The colorimetric reaction is stopped by addition of 100 μl of 1 M sulfuric acid and the absorbance in each well is read at 450 nm, using an ELISA plate reader (Model 3350-UV, BioRad).
With a correlation coefficient of 0.998 for the IgG standard curve, the following concentrations are determined for the four different culture concentrates (ca. 250-300 fold concentrated):

VHE/humV1 supernatant = 8.26 μg/ml
VHE/humV2 supernatant = 6.27 μg/ml
VHQ/humV1 supernatant = 5.3 μg/ml
VHQ/humV2 supernatant = 5.56 μg/ml

**Example 6: FACS competition analysis (binding affinity)**

The human T-cell line PEER is chosen as the target cell for FACS analysis because it expressed the CD45 antigen on its cell surface. To analyze the binding affinity of humanised antibody supernatants, competition experiments using FITC-labeled chimeric antibody as a reference are performed and compared with the inhibition of purified mouse antibody and of chimeric antibody. PEER cell cultures are centrifuged for 10 seconds at 3000 rpm and the medium is removed. Cells are resuspended in FACS buffer (PBS containing 1% FBS and 0.1% sodium azide) and seeded into 96-well round-bottom microtitter plate at a cell density of $1x10^5$ cells per well. The plate is centrifuged and the supernatant is discarded. For blocking studies, 25 μl of concentrated untransfected medium or isotype matched control antibody (negative controls), unlabeled mouse antibody or chimeric antibody (positive controls) as well as concentrated supernatant containing the various combinations of humanised antibody (samples), is first added in each well at the indicated concentrations in the text. After 1 hour of incubation at 4°C, PEER cells are washed with 200 μl of FACS buffer by centrifugation. Cells are subsequently incubated for 1 hour at 4°C with chimeric antibody conjugated with FITC in 25 μl of FACS buffer at the final concentration of 20 μg/ml. Cells are washed and resuspended in 300 μl of FACS buffer containing 2 μg/ml propidium iodide which allows gating of viable cells. The cell preparations are analyzed on a flow cytometer (FACSCalibur, Becton Dickinson).

FACS analysis indicates a dose-dependent blockade of fluorochrome-labeled chimeric antibody by the concentrated humanised antibody culture supernatants. No dose-dependent blockade of chimeric antibody binding is seen with the isotype matched control antibody, indicating that the blocking effect by the different humanised antibody combinations is epitope specific and that epitope specificity appears to be retained after the humanisation process.

**<u>Example 7:</u> Biological activities of CD45RB/RO binding molecules**

In this study, we have addressed whether CD45RB/RO binding chimeric antibody, when present in cultures of polyclonally activated primary human T cells (i) supports the differentiation of T cells with a characteristic Treg phenotype, (ii) prevents or enhances apoptosis following T cell activation, and (iii) affects expression of subset-specific antigens and receptors after restimulation.

**CD45RB/RO binding chimeric antibody enhances cell death in polyclonally activated T cells**

Primary T cells (mixture of CD4+ and CD8+ T subsets) were subjected to activation by anti-CD3 plus anti-CD28 mAb (200 ng/ml each) in the presence or absence (=control) of CD45RB/RO binding chimeric antibody. Excess antibodies were removed by washing on day 2. 7-amino-actinomycin D (7-AAD) as a DNA-staining dye taken up by apoptotic and necrotic cells was used to measure cell death following activation. The results show that activation of T cells in the presence of CD45RB/RO binding chimeric antibody increased the fraction of 7-AAD positive cells than two-fold on day 2 after activation.. On day 7, the portion of 7-AAD positive cells was again similar in CD45RB/RO binding chimeric antibody-treated and control cultures.

**CD45RB/RO binding chimeric antibody but not control mAb treated T cells display a T regulatory cell (Treg) phenotype**

Increased expression of CD25 and the negative regulatory protein CTLA-4 (CD152) is a marker of Treg cells. Functional suppression of primary and secondary T cell responses by CD45RB/RO binding chimeric antibody may be due to the induction of Treg cells. To address this issue, T cells were activated by anti-CD3 + CD28 mAbs and cultured in the presence of CD45RB/RO binding chimeric antibody or anti-LPS control mAb. The time address this issue, T cells were activated by anti-CD3 + CD28 mAbs and cultured in the presence of CD45RB/RO binding chimeric antibody or anti-LPS control mAb. The time course of CTLA-4 and CD25 expression reveals marked differences between controls and CD45RB/RO binding chimeric antibody-treated T cells on days 1 and 3 after secondary stimulation, indicating a Treg phenotype.

**Intracellular CTLA-4 expression is sustained in the presence of CD45RB/RO binding chimeric antibody**

It has been reported that substantial amounts of CTLA-4 can also be found intracellularly. Therefore, in parallel to surface CTLA-4 staining, intracellular CTLA-4 expression was analyzed. Moderate differences between T cell cultures were seen on day 4 after stimulation. After prolonged culture, however, high levels of intracellular CTLA-4 were sustained only in CD45RB/RO binding chimeric antibody-treated but not in control T cells.

**CD45RB/RO binding chimeric antibody -treated T cells become double positive for CD4 and CD8**

Following stimulation, T cells induce and upregulate the expression of several surface receptors, such as CD25, CD152 (CTLA-4), CD154 (CD40-Ligand) and others. In contrast, the level of expression of CD4 or CD8 is thought to stay relatively constant. We reproducibly observed a strong increase of both CD4 and CD8 antigens on CD45RB/RO binding chimeric antibody-treated but not on control Ab-treated T cells after activation. The emergence of a CD4/CD8 double-positive T cell population seems to be due to the upregulation of CD4 on the CD8+ subset and conversely, CD8 on the CD4+ subset. This contrasts with a moderately low percentage of double positive T cells in control cultures.

**High IL-2 receptor alpha-chain, but very low beta-chain expression by CD45RB/RO binding chimeric antibody-treated T cells**

Treg cells are known to be constitutively positive for CD25, the IL-2 receptor alpha-chain. The regulation of other subunits of the trimeric IL-2 receptor on Treg cells is not known. Recently we have compared the expression of the beta-chain of IL-2 receptor, e.g. CD122, on T cells activated and propagated in the presence or absence of CD45RB/RO binding chimeric antibody. The results show that CD45RB/RO binding chimeric antibody-treated T cells have about ten-fold lower CD122 expression as compared to T cells in control cultures. This difference may indicate that Treg cells require factors other than IL-2 to proliferate.

**Example 8: Sequences of the invention (CDR sequences of the invention are underlined)**

**SEQ ID NO:1**
**Part of the amino acid sequence of chimeric light chain**

DILLTQSPAILSVSPGERVSFSCRASQNIGTSIQWYQQRTNGSPRLLIRSSSESISGIPSRFSG
SGSGTDFTLSINSVESEDIADYYCQQSNTWPFTFGSGTKLEIK

**SEQ ID NO:2**
**Part of the amino acid sequence of chimeric heavy chain**

EVQLQQSGPELVKPGASVKMSCKASGYTFTNYIIHWVKQEPGQGLEWIGYFNPYNHGTKY
NEKFKGRATLTADKSSNTAYMDLSSLTSEDSAIYYCARSGPYAWFDTWGQGTTVTVSS

**SEQ ID NO:3**
**Amino acid sequence of chimeric light chain**

DILLTQSPAILSVSPGERVSFSCRASQNIGTSIQWYQQRTNGSPRLLIRSSSESISGIPSRFSG
SGSGTDFTLSINSVESEDIADYYCQQSNTWPFTFGSGTKLEIKRTVAAPSVFIFPPSDEQLKS
GTASVVCLLNNFYPREAKVQWKVDNALQSGNSQESVTEQDSKDSTYSLSSTLTLSKADYE
KHKVYACEVTHQGLSSPVTKSFNRGEC

**SEQ ID NO:4**
**Amino acid sequence of chimeric heavy chain**

EVQLQQSGPELVKPGASVKMSCKASGYTFTNYIIHWVKQEPGQGLEWIGYFNPYNHGTKY
NEKFKGRATLTADKSSNTAYMDLSSLTSEDSAIYYCARSGPYAWFDTWGQGTTVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSKLTVDKSRWQQGNVFS
CSVMHEALHNHYTQKSLSLSPGK

**SEQ ID NO:5**
**Nucleotide sequence encoding a polypeptide of SEQ ID NO:1**

GACATTCTGCTGACCCAGTCTCCAGCCATCCTGTCTGTGAGTCCAGGAGAAAGAGTCA
GTTTCTCCTGCAGGGCCAGTCAGAACATTGGCACAAGCATACAGTGGTATCAACAAAGA
ACAAATGGTTCTCCAAGGCTTCTCATAAGGTCTTCTTCTGAGTCTATCTCTGGGATCCCT
TCCAGGTTTAGTGGCAGTGGATCAGGGACAGATTTTACTCTTAGCATCAACAGTGTGGA
GTCTGAAGATATTGCAGATTATTACTGTCAACAAAGTAATACCTGGCCATTCACGTTCGG
CTCGGGGACCAAGCTTGAAATCAAA

**SEQ ID NO:6**
**Nucleotide sequence encoding a polypeptide of SEQ ID NO:2**

GAGGTGCAGCTGCAGCAGTCAGGACCTGAACTGGTAAAGCCTGGGGCTTCAGTGAAG
ATGTCCTGCAAGGCCTCTGGATACACATTCACTAATTATATTATCCACTGGGTGAAGCA
GGAGCCTGGTCAGGGCCTTGAATGGATTGGATATTTTAATCCTTACAATCATGGTACTA
AGTACAATGAGAAGTTCAAAGGCAGGGCCACACTAACTGCAGACAAATCCTCCAACACA
GCCTACATGGACCTCAGCAGCCTGACCTCTGAGGACTCTGCGATCTACTACTGTGCAA
GATCAGGACCCTATGCCTGGTTTGACACCTGGGGCCAAGGGACCACGGTCACCGTCTC
CTCA

**SEQ ID NO:7**
**Part of amino acid sequence of humanised light chain designated humV2 (humV2 =** VLm)

DILLTQSPAT LSLSPGERAT FSCRASQNIG TSIQWYQQKT NGAPRLLIRS SSESISGIPS
RFSGSGSGTD FTLTISSLEP EDFAVYYCQQ SNTWPFTFGQ GTKLEIK

**SEQ ID NO:8**
**Part of amino acid sequence of humanised light chain designated humV1 (humV1 = VLh)**

DILLTQSPAT LSLSPGERAT LSCRASQNIG TSIQWYQQKP GQAPRLLIRS SSESISGIPS RFSGSGSGTD FTLTISSLEP EDFAVYYCQQ SNTWPFTFGQ GTKLEIK

SEQ ID NO:9
**Part of amino acid sequence of humanised heavy chain designated VHE**

EVQLVESGAE VKKPGASVKV SCKASGYTFT NYIIHWVKQE PGQGLEWIGY FNPYNHGTKY NEKFKGRATL TANKSISTAY MELSSLRSED TAVYYCARSG PYAWFDTWGQ GTTVTVSS

SEQ ID NO:10
**Part of amino acid sequence of humanised heavy chain designated VHQ**

QVQLVESGAE VKKPGASVKV SCKASGYTFT NYIIHWVKQE PGQGLEWIGY FNPYNHGTKY NEKFKGRATL TANKSISTAY MELSSLRSED TAVYYCARSG PYAWFDTWGQ GTTVTVSS

SEQ ID NO:11
**Nucleotide sequence encoding amino acid sequence SEQ ID NO:9**

GAGGTGCAGCTGGTGGAGTCAGGAGCCGAAGTGAAAAAGCCTGGGGCTTCAGTGAAG
GTGTCCTGCAAGGCCTCTGGATACACATTCACTAATTATATTATCCACTGGGTGAAGCA
GGAGCCTGGTCAGGGCCTTGAATGGATTGGATATTTTAATCCTTACAATCATGGTACTA
AGTACAATGAGAAGTTCAAAGGCAGGGCCACACTAACTGCAAACAAATCCATCAGCACA
GCCTACATGGAGCTCAGCAGCCTGCGCTCTGAGGACACTGCGGTCTACTACTGTGCAA
GATCAGGACCCTATGCCTGGTTTGACACCTGGGGCCAAGGGACCACGGTCACCGTCTC
CTCA

SEQ ID NO:12
**Nucleotide sequence encoding amino acid sequence SEQ ID NO:10**

CAGGTGCAGCTGGTGGAGTCAGGAGCCGAAGTGAAAAAGCCTGGGGCTTCAGTGAAG
GTGTCCTGCAAGGCCTCTGGATACACATTCACTAATTATATTATCCACTGGGTGAAGCA
GGAGCCTGGTCAGGGCCTTGAATGGATTGGATATTTTAATCCTTACAATCATGGTACTA
AGTACAATGAGAAGTTCAAAGGCAGGGCCACACTAACTGCAAACAAATCCATCAGCACA
GCCTACATGGAGCTCAGCAGCCTGCGCTCTGAGGACACTGCGGTCTACTACTGTGCAA
GATCAGGACCCTATGCCTGGTTTGACACCTGGGGCCAAGGGACCACGGTCACCGTCTC
CTCA

**SEQ ID NO:13**
**Nucleotide sequence encoding amino acid sequence SEQ ID NO:7**

GACATTCTGCTGACCCAGTCTCCAGCCACCCTGTCTCTGAGTCCAGGAGAAAGAGCCA
CTTTCTCCTGCAGGGCCAGTCAGAACATTGGCACAAGCATACAGTGGTATCAACAAAAA
ACAAATGGTGCTCCAAGGCTTCTCATAAGGTCTTCTTCTGAGTCTATCTCTGGGATCCC
TTCCAGGTTTAGTGGCAGTGGATCAGGGACAGATTTTACTCTTACCATCAGCAGTCTGG
AGCCTGAAGATTTTGCAGTGTATTACTGTCAACAAAGTAATACCTGGCCATTCACGTTC
GGCCAGGGGACCAAGCTGGAGATCAAA

**SEQ ID NO:14**
**Nucleotide sequence encoding amino acid sequence SEQ ID NO:8**

GACATTCTGCTGACCCAGTCTCCAGCCACCCTGTCTCTGAGTCCAGGAGAAAGAGCCA
CTCTCTCCTGCAGGGCCAGTCAGAACATTGGCACAAGCATACAGTGGTATCAACAAAAA
CCAGGTCAGGCTCCAAGGCTTCTCATAAGGTCTTCTTCTGAGTCTATCTCTGGGATCCC
TTCCAGGTTTAGTGGCAGTGGATCAGGGACAGATTTTACTCTTACCATCAGCAGTCTGG
AGCCTGAAGATTTTGCAGTGTATTACTGTCAACAAAGTAATACCTGGCCATTCACGTTC
GGCCAGGGGACCAAGCTGGAGATCAAA

**SEQ ID NO:15**
**Nucleotide sequence of the expression vector HCMV-G1 HuAb-VHQ**
**(Complete DNA Sequence of a humanised heavy chain expression vector comprising SEQ ID NO:12 (VHQ) from 3921-4274)**

```
  1  AGCTTTTTGC AAAAGCCTAG GCCTCCAAAA AAGCCTCCTC ACTACTTCTG
 51  GAATAGCTCA GAGGCCGAGG CGGCCTCGGC CTCTGCATAA ATAAAAAAAA
101  TTAGTCAGCC ATGGGGCGGA GAATGGGCGG AACTGGGCGG AGTTAGGGGC
151  GGGATGGGCG GAGTTAGGGG CGGGACTATG GTTGCTGACT AATTGAGATG
201  CATGCTTTGC ATACTTCTGC CTGCTGGGGA GCCTGGTTGC TGACTAATTG
251  AGATGCATGC TTTGCATACT TCTGCCTGCT GGGGAGCCTG GGGACTTTCC
301  ACACCCTAAC TGACACACAT TCCACAGCTG CCTCGCGCGT TTCGGTGATG
351  ACGGTGAAAA CCTCTGACAC ATGCAGCTCC CGGAGACGGT CACAGCTTGT
```

401 CTGTAAGCGG ATGCCGGGAG CAGACAAGCC CGTCAGGGCG CGTCAGCGGG
451 TGTTGGCGGG TGTCGGGGCG CAGCCATGAC CCAGTCACGT AGCGATAGCG
501 GAGTGTATAC TGGCTTAACT ATGCGGCATC AGAGCAGATT GTACTGAGAG
551 TGCACCATAT GCGGTGTGAA ATACCGCACA GATGCGTAAG GAGAAAATAC
601 CGCATCAGGC GCTCTTCCGC TTCCTCGCTC ACTGACTCGC TGCGCTCGGT
651 CGTTCGGCTG CGGCGAGCGG TATCAGCTCA CTCAAAGGCG GTAATACGGT
701 TATCCACAGA ATCAGGGGAT AACGCAGGAA AGAACATGTG AGCAAAAGGC
751 CAGCAAAAGG CCAGGAACCG TAAAAAGGCC GCGTTGCTGG CGTTTTTCCA
801 TAGGCTCCGC CCCCCTGACG AGCATCACAA AAATCGACGC TCAAGTCAGA
851 GGTGGCGAAA CCCGACAGGA CTATAAAGAT ACCAGGCGTT TCCCCCTGGA
901 AGCTCCCTCG TGCGCTCTCC TGTTCCGACC CTGCCGCTTA CCGGATACCT
951 GTCCGCCTTT CTCCCTTCGG GAAGCGTGGC GCTTTCTCAT AGCTCACGCT
1001 GTAGGTATCT CAGTTCGGTG TAGGTCGTTC GCTCCAAGCT GGGCTGTGTG
1051 CACGAACCCC CCGTTCAGCC CGACCGCTGC GCCTTATCCG GTAACTATCG
1101 TCTTGAGTCC AACCCGGTAA GACACGACTT ATCGCCACTG GCAGCAGCCA
1151 CTGGTAACAG GATTAGCAGA GCGAGGTATG TAGGCGGTGC TACAGAGTTC
1201 TTGAAGTGGT GGCCTAACTA CGGCTACACT AGAAGGACAG TATTTGGTAT
1251 CTGCGCTCTG CTGAAGCCAG TTACCTTCGG AAAAAGAGTT GGTAGCTCTT
1301 GATCCGGCAA ACAAACCACC GCTGGTAGCG GTGGTTTTTT TGTTTGCAAG
1351 CAGCAGATTA CGCGCAGAAA AAAAGGATCT CAAGAAGATC CTTTGATCTT
1401 TTCTACGGGG TCTGACGCTC AGTGGAACGA AAACTCACGT TAAGGGATTT
1451 TGGTCATGAG ATTATCAAAA AGGATCTTCA CCTAGATCCT TTTAAATTAA
1501 AAATGAAGTT TTAAATCAAT CTAAAGTATA TATGAGTAAA CTTGGTCTGA
1551 CAGTTACCAA TGCTTAATCA GTGAGGCACC TATCTCAGCG ATCTGTCTAT
1601 TTCGTTCATC CATAGTTGCC TGACTCCCCG TCGTGTAGAT AACTACGATA
1651 CGGGAGGGCT TACCATCTGG CCCCAGTGCT GCAATGATAC CGCGAGACCC
1701 ACGCTCACCG GCTCCAGATT TATCAGCAAT AAACCAGCCA GCCGGAAGGG
1751 CCGAGCGCAG AAGTGGTCCT GCAACTTTAT CCGCCTCCAT CCAGTCTATT
1801 AATTGTTGCC GGGAAGCTAG AGTAAGTAGT TCGCCAGTTA ATAGTTTGCG
1851 CAACGTTGTT GCCATTGCTG CAGGCATCGT GGTGTCACGC TCGTCGTTTG
1901 GTATGGCTTC ATTCAGCTCC GGTTCCCAAC GATCAAGGCG AGTTACATGA
1951 TCCCCCATGT TGTGCAAAAA AGCGGTTAGC TCCTTCGGTC CTCCGATCGT
2001 TGTCAGAAGT AAGTTGGCCG CAGTGTTATC ACTCATGGTT ATGGCAGCAC

2051 TGCATAATTC TCTTACTGTC ATGCCATCCG TAAGATGCTT TTCTGTGACT
2101 GGTGAGTACT CAACCAAGTC ATTCTGAGAA TAGTGTATGC GGCGACCGAG
2151 TTGCTCTTGC CCGGCGTCAA CACGGGATAA TACCGCGCCA CATAGCAGAA
2201 CTTTAAAAGT GCTCATCATT GGAAAACGTT CTTCGGGGCG AAAACTCTCA
2251 AGGATCTTAC CGCTGTTGAG ATCCAGTTCG ATGTAACCCA CTCGTGCACC
2301 CAACTGATCT TCAGCATCTT TTACTTTCAC CAGCGTTTCT GGGTGAGCAA
2351 AAACAGGAAG GCAAAATGCC GCAAAAAAGG GAATAAGGGC GACACGGAAA
2401 TGTTGAATAC TCATACTCTT CCTTTTTCAA TATTATTGAA GCATTTATCA
2451 GGGTTATTGT CTCATGAGCG GATACATATT TGAATGTATT TAGAAAAATA
2501 AACAAATAGG GGTTCCGCGC ACATTTCCCC GAAAAGTGCC ACCTGACGTC
2551 TAAGAAACCA TTATTATCAT GACATTAACC TATAAAAATA GGCGTATCAC
2601 GAGGCCCTTT CGTCTTCAAG AATTCAGCTT GGCTGCAGTG AATAATAAAA
2651 TGTGTGTTTG TCCGAAATAC GCGTTTTGAG ATTTCTGTCG CCGACTAAAT
2701 TCATGTCGCG CGATAGTGGT GTTTATCGCC GATAGAGATG GCGATATTGG
2751 AAAAATCGAT ATTTGAAAAT ATGGCATATT GAAAATGTCG CCGATGTGAG
2801 TTTCTGTGTA ACTGATATCG CCATTTTTCC AAAAGTGATT TTTGGGCATA
2851 CGCGATATCT GGCGATAGCG CTTATATCGT TTACGGGGGA TGGCGATAGA
2901 CGACTTTGGT GACTTGGGCG ATTCTGTGTG TCGCAAATAT CGCAGTTTCG
2951 ATATAGGTGA CAGACGATAT GAGGCTATAT CGCCGATAGA GGCGACATCA
3001 AGCTGGCACA TGGCCAATGC ATATCGATCT ATACATTGAA TCAATATTGG
3051 CCATTAGCCA TATTATTCAT TGGTTATATA GCATAAATCA ATATTGGCTA
3101 TTGGCCATTG CATACGTTGT ATCCATATCA TAATATGTAC ATTTATATTG
3151 GCTCATGTCC AACATTACCG CCATGTTGAC ATTGATTATT GACTAGTTAT
3201 TAATAGTAAT CAATTACGGG GTCATTAGTT CATAGCCCAT ATATGGAGTT
3251 CCGCGTTACA TAACTTACGG TAAATGGCCC GCCTGGCTGA CCGCCCAACG
3301 ACCCCCGCCC ATTGACGTCA ATAATGACGT ATGTTCCCAT AGTAACGCCA
3351 ATAGGGACTT TCCATTGACG TCAATGGGTG GAGTATTTAC GGTAAACTGC
3401 CCACTTGGCA GTACATCAAG TGTATCATAT GCCAAGTACG CCCCCTATTG
3451 ACGTCAATGA CGGTAAATGG CCCGCCTGGC ATTATGCCCA GTACATGACC
3501 TTATGGGACT TTCCTACTTG GCAGTACATC TACGTATTAG TCATCGCTAT
3551 TACCATGGTG ATGCGGTTTT GGCAGTACAT CAATGGGCGT GGATAGCGGT
3601 TTGACTCACG GGGATTTCCA AGTCTCCACC CCATTGACGT CAATGGGAGT
3651 TTGTTTTGGC ACCAAAATCA ACGGGACTTT CCAAAATGTC GTAACAACTC

3701 CGCCCCATTG ACGCAAATGG GCGGTAGGCG TGTACGGTGG GAGGTCTATA
3751 TAAGCAGAGC TCGTTTAGTG AACCGTCAGA TCGCCTGGAG ACGCCATCCA
3801 CGCTGTTTTG ACCTCCATAG AAGACACCGG GACCGATCCA GCCTCCGCAA
3851 GCTTGCCGCC ACCATGGACT GGACCTGGAG GGTGTTCTGC CTGCTGGCCG
3901 TGGCCCCCGG CGCCCACAGC CAGGTGCAGC TGGTGGAGTC AGGAGCCGAA
3951 GTGAAAAAGC CTGGGGCTTC AGTGAAGGTG TCCTGCAAGG CCTCTGGATA
4001 CACATTCACT AATTATATTA TCCACTGGGT GAAGCAGGAG CCTGGTCAGG
4051 GCCTTGAATG GATTGGATAT TTTAATCCTT ACAATCATGG TACTAAGTAC
4101 AATGAGAAGT TCAAAGGCAG GGCCACACTA ACTGCAAACA AATCCATCAG
4151 CACAGCCTAC ATGGAGCTCA GCAGCCTGCG CTCTGAGGAC ACTGCGGTCT
4201 ACTACTGTGC AAGATCAGGA CCCTATGCCT GGTTTGACAC CTGGGGCCAA
4251 GGGACCACGG TCACCGTCTC CTCAGGTGAG TTCTAGAAGG ATCCCAAGCT
4301 AGCTTTCTGG GGCAGGCCAG GCCTGACCTT GGCTTTGGGG CAGGGAGGGG
4351 GCTAAGGTGA GGCAGGTGGC GCCAGCCAGG TGCACACCCA ATGCCCATGA
4401 GCCCAGACAC TGGACGCTGA ACCTCGCGGA CAGTTAAGAA CCCAGGGGCC
4451 TCTGCGCCCT GGGCCCAGCT CTGTCCCACA CCGCGGTCAC ATGGCACCAC
4501 CTCTCTTGCA GCCTCCACCA AGGGCCCATC GGTCTTCCCC CTGGCACCCT
4551 CCTCCAAGAG CACCTCTGGG GGCACAGCGG CCCTGGGCTG CCTGGTCAAG
4601 GACTACTTCC CCGAACCGGT GACGGTGTCG TGGAACTCAG GCGCCCTGAC
4651 CAGCGGCGTG CACACCTTCC CGGCTGTCCT ACAGTCCTCA GGACTCTACT
4701 CCCTCAGCAG CGTGGTGACC GTGCCCTCCA GCAGCTTGGG CACCCAGACC
4751 TACATCTGCA ACGTGAATCA CAAGCCCAGC AACACCAAGG TGGACAAGAA
4801 AGTTGGTGAG AGGCCAGCAC AGGGAGGGAG GGTGTCTGCT GGAAGCCAGG
4851 CTCAGCGCTC CTGCCTGGAC GCATCCCGGC TATGCAGCCC CAGTCCAGGG
4901 CAGCAAGGCA GGCCCCGTCT GCCTCTTCAC CCGGAGGCCT CTGCCCGCCC
4951 CACTCATGCT CAGGGAGAGG GTCTTCTGGC TTTTTCCCCA GGCTCTGGGC
5001 AGGCACAGGC TAGGTGCCCC TAACCCAGGC CCTGCACACA AAGGGGCAGG
5051 TGCTGGGCTC AGACCTGCCA AGAGCCATAT CCGGGAGGAC CCTGCCCCTG
5101 ACCTAAGCCC ACCCCAAAGG CCAAACTCTC CACTCCCTCA GCTCGGACAC
5151 CTTCTCTCCT CCCAGATTCC AGTAACTCCC AATCTTCTCT CTGCAGAGCC
5201 CAAATCTTGT GACAAAACTC ACACATGCCC ACCGTGCCCA GGTAAGCCAG
5251 CCCAGGCCTC GCCCTCCAGC TCAAGGCGGG ACAGGTGCCC TAGAGTAGCC
5301 TGCATCCAGG GACAGGCCCC AGCCGGGTGC TGACACGTCC ACCTCCATCT

5351 CTTCCTCAGC ACCTGAACTC CTGGGGGGAC CGTCAGTCTT CCTCTTCCCC
5401 CCAAAACCCA AGGACACCCT CATGATCTCC CGGACCCCTG AGGTCACATG
5451 CGTGGTGGTG GACGTGAGCC ACGAAGACCC TGAGGTCAAG TTCAACTGGT
5501 ACGTGGACGG CGTGGAGGTG CATAATGCCA AGACAAAGCC GCGGGAGGAG
5551 CAGTACAACA GCACGTACCG TGTGGTCAGC GTCCTCACCG TCCTGCACCA
5601 GGACTGGCTG AATGGCAAGG AGTACAAGTG CAAGGTCTCC AACAAAGCCC
5651 TCCCAGCCCC CATCGAGAAA ACCATCTCCA AAGCCAAAGG TGGGACCCGT
5701 GGGGTGCGAG GGCCACATGG ACAGAGGCCG GCTCGGCCCA CCCTCTGCCC
5751 TGAGAGTGAC CGCTGTACCA ACCTCTGTCC CTACAGGGCA GCCCCGAGAA
5801 CCACAGGTGT ACACCCTGCC CCCATCCCGG GATGAGCTGA CCAAGAACCA
5851 GGTCAGCCTG ACCTGCCTGG TCAAAGGCTT CTATCCCAGC GACATCGCCG
5901 TGGAGTGGGA GAGCAATGGG CAGCCGGAGA ACAACTACAA GACCACGCCT
5951 CCCGTGCTGG ACTCCGACGG CTCCTTCTTC CTCTACAGCA AGCTCACCGT
6001 GGACAAGAGC AGGTGGCAGC AGGGGAACGT CTTCTCATGC TCCGTGATGC
6051 ATGAGGCTCT GCACAACCAC TACACGCAGA AGAGCCTCTC CCTGTCTCCG
6101 GGTAAATGAG TGCGACGGCC GGCAAGCCCC CGCTCCCCGG GCTCTCGCGG
6151 TCGCACGAGG ATGCTTGGCA CGTACCCCCT GTACATACTT CCCGGGCGCC
6201 CAGCATGGAA ATAAAGCACC CAGCGCTGCC CTGGGCCCCT GCGAGACTGT
6251 GATGGTTCTT TCCACGGGTC AGGCCGAGTC TGAGGCCTGA GTGGCATGAG
6301 ATCTGATATC ATCGATGAAT TCGAGCTCGG TACCCGGGGA TCGATCCAGA
6351 CATGATAAGA TACATTGATG AGTTTGGACA AACCACAACT AGAATGCAGT
6401 GAAAAAAATG CTTTATTTGT GAAATTTGTG ATGCTATTGC TTTATTTGTA
6451 ACCATTATAA GCTGCAATAA ACAAGTTAAC AACAACAATT GCATTCATTT
6501 TATGTTTCAG GTTCAGGGGG AGGTGTGGGA GGTTTTTTAA AGCAAGTAAA
6551 ACCTCTACAA ATGTGGTATG GCTGATTATG ATCTCTAGTC AAGGCACTAT
6601 ACATCAAATA TTCCTTATTA ACCCCTTTAC AAATTAAAAA GCTAAAGGTA
6651 CACAATTTTT GAGCATAGTT ATTAATAGCA GACACTCTAT GCCTGTGTGG
6701 AGTAAGAAAA AACAGTATGT TATGATTATA ACTGTTATGC CTACTTATAA
6751 AGGTTACAGA ATATTTTTCC ATAATTTTCT TGTATAGCAG TGCAGCTTTT
6801 TCCTTTGTGG TGTAAATAGC AAAGCAAGCA AGAGTTCTAT TACTAAACAC
6851 AGCATGACTC AAAAAACTTA GCAATTCTGA AGGAAAGTCC TTGGGGTCTT
6901 CTACCTTTCT CTTCTTTTTT GGAGGAGTAG AATGTTGAGA GTCAGCAGTA
6951 GCCTCATCAT CACTAGATGG CATTTCTTCT GAGCAAAACA GGTTTTCCTC

7001 ATTAAAGGCA TTCCACCACT GCTCCCATTC ATCAGTTCCA TAGGTTGGAA
7051 TCTAAAATAC ACAAACAATT AGAATCAGTA GTTTAACACA TTATACACTT
7101 AAAAATTTTA TATTTACCTT AGAGCTTTAA ATCTCTGTAG GTAGTTTGTC
7151 CAATTATGTC ACACCACAGA AGTAAGGTTC CTTCACAAAG ATCCGGGACC
7201 AAAGCGGCCA TCGTGCCTCC CCACTCCTGC AGTTCGGGGG CATGGATGCG
7251 CGGATAGCCG CTGCTGGTTT CCTGGATGCC GACGGATTTG CACTGCCGGT
7301 AGAACTCCGC GAGGTCGTCC AGCCTCAGGC AGCAGCTGAA CCAACTCGCG
7351 AGGGGATCGA GCCCGGGGTG GGCGAAGAAC TCCAGCATGA GATCCCCGCG
7401 CTGGAGGATC ATCCAGCCGG CGTCCCGGAA AACGATTCCG AAGCCCAACC
7451 TTTCATAGAA GGCGGCGGTG GAATCGAAAT CTCGTGATGG CAGGTTGGGC
7501 GTCGCTTGGT CGGTCATTTC GAACCCCAGA GTCCCGCTCA GAAGAACTCG
7551 TCAAGAAGGC GATAGAAGGC GATGCGCTGC GAATCGGGAG CGGCGATACC
7601 GTAAAGCACG AGGAAGCGGT CAGCCCATTC GCCGCCAAGC TCTTCAGCAA
7651 TATCACGGGT AGCCAACGCT ATGTCCTGAT AGCGGTCCGC CACACCCAGC
7701 CGGCCACAGT CGATGAATCC AGAAAAGCGG CCATTTTCCA CCATGATATT
7751 CGGCAAGCAG GCATCGCCAT GGGTCACGAC GAGATCCTCG CCGTCGGGCA
7801 TGCGCGCCTT GAGCCTGGCG AACAGTTCGG CTGGCGCGAG CCCCTGATGC
7851 TCTTCGTCCA GATCATCCTG ATCGACAAGA CCGGCTTCCA TCCGAGTACG
7901 TGCTCGCTCG ATGCGATGTT TCGCTTGGTG GTCGAATGGG CAGGTAGCCG
7951 GATCAAGCGT ATGCAGCCGC CGCATTGCAT CAGCCATGAT GGATACTTTC
8001 TCGGCAGGAG CAAGGTGAGA TGACAGGAGA TCCTGCCCCG GCACTTCGCC
8051 CAATAGCAGC CAGTCCCTTC CCGCTTCAGT GACAACGTCG AGCACAGCTG
8101 CGCAAGGAAC GCCCGTCGTG GCCAGCCACG ATAGCCGCGC TGCCTCGTCC
8151 TGCAGTTCAT TCAGGGCACC GGACAGGTCG GTCTTGACAA AAAGAACCGG
8201 GCGCCCCTGC GCTGACAGCC GGAACACGGC GGCATCAGAG CAGCCGATTG
8251 TCTGTTGTGC CCAGTCATAG CCGAATAGCC TCTCCACCCA AGCGGCCGGA
8301 GAACCTGCGT GCAATCCATC TTGTTCAATC ATGCGAAACG ATCCTCATCC
8351 TGTCTCTTGA TCAGATCTTG ATCCCCTGCG CCATCAGATC CTTGGCGGCA
8401 AGAAAGCCAT CCAGTTTACT TTGCAGGGCT TCCCAACCTT ACCAGAGGGC
8451 GCCCCAGCTG GCAATTCCGG TTCGCTTGCT GTCCATAAAA CCGCCCAGTC
8501 TAGCTATCGC CATGTAAGCC CACTGCAAGC TACCTGCTTT CTCTTTGCGC
8551 TTGCGTTTTC CCTTGTCCAG ATAGCCCAGT AGCTGACATT CATCCGGGGT
8601 CAGCACCGTT TCTGCGGACT GGCTTTCTAC GTGTTCCGCT TCCTTTAGCA

8651 GCCCTTGCGC CCTGAGTGCT TGCGGCAGCG TGAAGCT

SEQ ID NO:16

**Nucleotide sequence of the expression vector HCMV-G1 HuAb-VHE**

**(Complete DNA Sequence of a humanised heavy chain expression vector comprising SEQ ID NO: 11 (VHE) from 3921-4274)**

1 AGCTTTTTGC AAAAGCCTAG GCCTCCAAAA AAGCCTCCTC ACTACTTCTG
51 GAATAGCTCA GAGGCCGAGG CGGCCTCGGC CTCTGCATAA ATAAAAAAAA
101 TTAGTCAGCC ATGGGGCGGA GAATGGGCGG AACTGGGCGG AGTTAGGGGC
151 GGGATGGGCG GAGTTAGGGG CGGGACTATG GTTGCTGACT AATTGAGATG
201 CATGCTTTGC ATACTTCTGC CTGCTGGGGA GCCTGGTTGC TGACTAATTG
251 AGATGCATGC TTTGCATACT TCTGCCTGCT GGGGAGCCTG GGGACTTTCC
301 ACACCCTAAC TGACACACAT TCCACAGCTG CCTCGCGCGT TTCGGTGATG
351 ACGGTGAAAA CCTCTGACAC ATGCAGCTCC CGGAGACGGT CACAGCTTGT
401 CTGTAAGCGG ATGCCGGGAG CAGACAAGCC CGTCAGGGCG CGTCAGCGGG
451 TGTTGGCGGG TGTCGGGGCG CAGCCATGAC CCAGTCACGT AGCGATAGCG
501 GAGTGTATAC TGGCTTAACT ATGCGGCATC AGAGCAGATT GTACTGAGAG
551 TGCACCATAT GCGGTGTGAA ATACCGCACA GATGCGTAAG GAGAAAATAC
601 CGCATCAGGC GCTCTTCCGC TTCCTCGCTC ACTGACTCGC TGCGCTCGGT
651 CGTTCGGCTG CGGCGAGCGG TATCAGCTCA CTCAAAGGCG GTAATACGGT
701 TATCCACAGA ATCAGGGGAT AACGCAGGAA AGAACATGTG AGCAAAAGGC
751 CAGCAAAAGG CCAGGAACCG TAAAAAGGCC GCGTTGCTGG CGTTTTTCCA
801 TAGGCTCCGC CCCCCTGACG AGCATCACAA AAATCGACGC TCAAGTCAGA
851 GGTGGCGAAA CCCGACAGGA CTATAAAGAT ACCAGGCGTT TCCCCCTGGA
901 AGCTCCCTCG TGCGCTCTCC TGTTCCGACC CTGCCGCTTA CCGGATACCT
951 GTCCGCCTTT CTCCCTTCGG GAAGCGTGGC GCTTTCTCAT AGCTCACGCT
1001 GTAGGTATCT CAGTTCGGTG TAGGTCGTTC GCTCCAAGCT GGGCTGTGTG
1051 CACGAACCCC CCGTTCAGCC CGACCGCTGC GCCTTATCCG GTAACTATCG
1101 TCTTGAGTCC AACCCGGTAA GACACGACTT ATCGCCACTG GCAGCAGCCA
1151 CTGGTAACAG GATTAGCAGA GCGAGGTATG TAGGCGGTGC TACAGAGTTC
1201 TTGAAGTGGT GGCCTAACTA CGGCTACACT AGAAGGACAG TATTTGGTAT
1251 CTGCGCTCTG CTGAAGCCAG TTACCTTCGG AAAAAGAGTT GGTAGCTCTT

1301 GATCCGGCAA ACAAACCACC GCTGGTAGCG GTGGTTTTTT TGTTTGCAAG
1351 CAGCAGATTA CGCGCAGAAA AAAAGGATCT CAAGAAGATC CTTTGATCTT
1401 TTCTACGGGG TCTGACGCTC AGTGGAACGA AAACTCACGT TAAGGGATTT
1451 TGGTCATGAG ATTATCAAAA AGGATCTTCA CCTAGATCCT TTTAAATTAA
1501 AAATGAAGTT TTAAATCAAT CTAAAGTATA TATGAGTAAA CTTGGTCTGA
1551 CAGTTACCAA TGCTTAATCA GTGAGGCACC TATCTCAGCG ATCTGTCTAT
1601 TTCGTTCATC CATAGTTGCC TGACTCCCCG TCGTGTAGAT AACTACGATA
1651 CGGGAGGGCT TACCATCTGG CCCCAGTGCT GCAATGATAC CGCGAGACCC
1701 ACGCTCACCG GCTCCAGATT TATCAGCAAT AAACCAGCCA GCCGGAAGGG
1751 CCGAGCGCAG AAGTGGTCCT GCAACTTTAT CCGCCTCCAT CCAGTCTATT
1801 AATTGTTGCC GGGAAGCTAG AGTAAGTAGT TCGCCAGTTA ATAGTTTGCG
1851 CAACGTTGTT GCCATTGCTG CAGGCATCGT GGTGTCACGC TCGTCGTTTG
1901 GTATGGCTTC ATTCAGCTCC GGTTCCCAAC GATCAAGGCG AGTTACATGA
1951 TCCCCCATGT TGTGCAAAAA AGCGGTTAGC TCCTTCGGTC CTCCGATCGT
2001 TGTCAGAAGT AAGTTGGCCG CAGTGTTATC ACTCATGGTT ATGGCAGCAC
2051 TGCATAATTC TCTTACTGTC ATGCCATCCG TAAGATGCTT TTCTGTGACT
2101 GGTGAGTACT CAACCAAGTC ATTCTGAGAA TAGTGTATGC GGCGACCGAG
2151 TTGCTCTTGC CCGGCGTCAA CACGGGATAA TACCGCGCCA CATAGCAGAA
2201 CTTTAAAAGT GCTCATCATT GGAAAACGTT CTTCGGGGCG AAAACTCTCA
2251 AGGATCTTAC CGCTGTTGAG ATCCAGTTCG ATGTAACCCA CTCGTGCACC
2301 CAACTGATCT TCAGCATCTT TTACTTTCAC CAGCGTTTCT GGGTGAGCAA
2351 AAACAGGAAG GCAAAATGCC GCAAAAAAGG GAATAAGGGC GACACGGAAA
2401 TGTTGAATAC TCATACTCTT CCTTTTTCAA TATTATTGAA GCATTTATCA
2451 GGGTTATTGT CTCATGAGCG GATACATATT TGAATGTATT TAGAAAAATA
2501 AACAAATAGG GGTTCCGCGC ACATTTCCCC GAAAAGTGCC ACCTGACGTC
2551 TAAGAAACCA TTATTATCAT GACATTAACC TATAAAAATA GGCGTATCAC
2601 GAGGCCCTTT CGTCTTCAAG AATTCAGCTT GGCTGCAGTG AATAATAAAA
2651 TGTGTGTTTG TCCGAAATAC GCGTTTTGAG ATTTCTGTCG CCGACTAAAT
2701 TCATGTCGCG CGATAGTGGT GTTTATCGCC GATAGAGATG GCGATATTGG
2751 AAAAATCGAT ATTTGAAAAT ATGGCATATT GAAAATGTCG CCGATGTGAG
2801 TTTCTGTGTA ACTGATATCG CCATTTTTCC AAAAGTGATT TTTGGGCATA
2851 CGCGATATCT GGCGATAGCG CTTATATCGT TTACGGGGGA TGGCGATAGA
2901 CGACTTTGGT GACTTGGGCG ATTCTGTGTG TCGCAAATAT CGCAGTTTCG

```
2951  ATATAGGTGA CAGACGATAT GAGGCTATAT CGCCGATAGA GGCGACATCA
3001  AGCTGGCACA TGGCCAATGC ATATCGATCT ATACATTGAA TCAATATTGG
3051  CCATTAGCCA TATTATTCAT TGGTTATATA GCATAAATCA ATATTGGCTA
3101  TTGGCCATTG CATACGTTGT ATCCATATCA TAATATGTAC ATTTATATTG
3151  GCTCATGTCC AACATTACCG CCATGTTGAC ATTGATTATT GACTAGTTAT
3201  TAATAGTAAT CAATTACGGG GTCATTAGTT CATAGCCCAT ATATGGAGTT
3251  CCGCGTTACA TAACTTACGG TAAATGGCCC GCCTGGCTGA CCGCCCAACG
3301  ACCCCCGCCC ATTGACGTCA ATAATGACGT ATGTTCCCAT AGTAACGCCA
3351  ATAGGGACTT TCCATTGACG TCAATGGGTG GAGTATTTAC GGTAAACTGC
3401  CCACTTGGCA GTACATCAAG TGTATCATAT GCCAAGTACG CCCCCTATTG
3451  ACGTCAATGA CGGTAAATGG CCCGCCTGGC ATTATGCCCA GTACATGACC
3501  TTATGGGACT TTCCTACTTG GCAGTACATC TACGTATTAG TCATCGCTAT
3551  TACCATGGTG ATGCGGTTTT GGCAGTACAT CAATGGGCGT GGATAGCGGT
3601  TTGACTCACG GGGATTTCCA AGTCTCCACC CCATTGACGT CAATGGGAGT
3651  TTGTTTTGGC ACCAAAATCA ACGGGACTTT CCAAAATGTC GTAACAACTC
3701  CGCCCCATTG ACGCAAATGG GCGGTAGGCG TGTACGGTGG GAGGTCTATA
3751  TAAGCAGAGC TCGTTTAGTG AACCGTCAGA TCGCCTGGAG ACGCCATCCA
3801  CGCTGTTTTG ACCTCCATAG AAGACACCGG GACCGATCCA GCCTCCGCAA
3851  GCTTGCCGCC ACCATGGACT GGACCTGGAG GGTGTTCTGC CTGCTGGCCG
3901  TGGCCCCCGG CGCCCACAGC GAGGTGCAGC TGGTGGAGTC AGGAGCCGAA
3951  GTGAAAAAGC CTGGGGCTTC AGTGAAGGTG TCCTGCAAGG CCTCTGGATA
4001  CACATTCACT AATTATATTA TCCACTGGGT GAAGCAGGAG CCTGGTCAGG
4051  GCCTTGAATG GATTGGATAT TTTAATCCTT ACAATCATGG TACTAAGTAC
4101  AATGAGAAGT TCAAAGGCAG GGCCACACTA ACTGCAAACA AATCCATCAG
4151  CACAGCCTAC ATGGAGCTCA GCAGCCTGCG CTCTGAGGAC ACTGCGGTCT
4201  ACTACTGTGC AAGATCAGGA CCCTATGCCT GGTTTGACAC CTGGGGCCAA
4251  GGGACCACGG TCACCGTCTC CTCAGGTGAG TTCTAGAAGG ATCCCAAGCT
4301  AGCTTTCTGG GGCAGGCCAG GCCTGACCTT GGCTTTGGGG CAGGGAGGGG
4351  GCTAAGGTGA GGCAGGTGGC GCCAGCCAGG TGCACACCCA ATGCCCATGA
4401  GCCCAGACAC TGGACGCTGA ACCTCGCGGA CAGTTAAGAA CCCAGGGGCC
4451  TCTGCGCCCT GGGCCCAGCT CTGTCCCACA CCGCGGTCAC ATGGCACCAC
4501  CTCTCTTGCA GCCTCCACCA AGGGCCCATC GGTCTTCCCC CTGGCACCCT
4551  CCTCCAAGAG CACCTCTGGG GGCACAGCGG CCCTGGGCTG CCTGGTCAAG
```

4601 GACTACTTCC CCGAACCGGT GACGGTGTCG TGGAACTCAG GCGCCCTGAC
4651 CAGCGGCGTG CACACCTTCC CGGCTGTCCT ACAGTCCTCA GGACTCTACT
4701 CCCTCAGCAG CGTGGTGACC GTGCCCTCCA GCAGCTTGGG CACCCAGACC
4751 TACATCTGCA ACGTGAATCA CAAGCCCAGC AACACCAAGG TGGACAAGAA
4801 AGTTGGTGAG AGGCCAGCAC AGGGAGGGAG GGTGTCTGCT GGAAGCCAGG
4851 CTCAGCGCTC CTGCCTGGAC GCATCCCGGC TATGCAGCCC CAGTCCAGGG
4901 CAGCAAGGCA GGCCCCGTCT GCCTCTTCAC CCGGAGGCCT CTGCCCGCCC
4951 CACTCATGCT CAGGGAGAGG GTCTTCTGGC TTTTTCCCCA GGCTCTGGGC
5001 AGGCACAGGC TAGGTGCCCC TAACCCAGGC CCTGCACACA AAGGGGCAGG
5051 TGCTGGGCTC AGACCTGCCA AGAGCCATAT CCGGGAGGAC CCTGCCCCTG
5101 ACCTAAGCCC ACCCCAAAGG CCAAACTCTC CACTCCCTCA GCTCGGACAC
5151 CTTCTCTCCT CCCAGATTCC AGTAACTCCC AATCTTCTCT CTGCAGAGCC
5201 CAAATCTTGT GACAAAACTC ACACATGCCC ACCGTGCCCA GGTAAGCCAG
5251 CCCAGGCCTC GCCCTCCAGC TCAAGGCGGG ACAGGTGCCC TAGAGTAGCC
5301 TGCATCCAGG GACAGGCCCC AGCCGGGTGC TGACACGTCC ACCTCCATCT
5351 CTTCCTCAGC ACCTGAACTC CTGGGGGGAC CGTCAGTCTT CCTCTTCCCC
5401 CCAAAACCCA AGGACACCCT CATGATCTCC CGGACCCCTG AGGTCACATG
5451 CGTGGTGGTG GACGTGAGCC ACGAAGACCC TGAGGTCAAG TTCAACTGGT
5501 ACGTGGACGG CGTGGAGGTG CATAATGCCA AGACAAAGCC GCGGGAGGAG
5551 CAGTACAACA GCACGTACCG TGTGGTCAGC GTCCTCACCG TCCTGCACCA
5601 GGACTGGCTG AATGGCAAGG AGTACAAGTG CAAGGTCTCC AACAAAGCCC
5651 TCCCAGCCCC CATCGAGAAA ACCATCTCCA AAGCCAAAGG TGGGACCCGT
5701 GGGGTGCGAG GGCCACATGG ACAGAGGCCG GCTCGGCCCA CCCTCTGCCC
5751 TGAGAGTGAC CGCTGTACCA ACCTCTGTCC CTACAGGGCA GCCCCGAGAA
5801 CCACAGGTGT ACACCCTGCC CCCATCCCGG GATGAGCTGA CCAAGAACCA
5851 GGTCAGCCTG ACCTGCCTGG TCAAAGGCTT CTATCCCAGC GACATCGCCG
5901 TGGAGTGGGA GAGCAATGGG CAGCCGGAGA ACAACTACAA GACCACGCCT
5951 CCCGTGCTGG ACTCCGACGG CTCCTTCTTC CTCTACAGCA AGCTCACCGT
6001 GGACAAGAGC AGGTGGCAGC AGGGGAACGT CTTCTCATGC TCCGTGATGC
6051 ATGAGGCTCT GCACAACCAC TACACGCAGA AGAGCCTCTC CCTGTCTCCG
6101 GGTAAATGAG TGCGACGGCC GGCAAGCCCC CGCTCCCCGG GCTCTCGCGG
6151 TCGCACGAGG ATGCTTGGCA CGTACCCCCT GTACATACTT CCCGGGCGCC
6201 CAGCATGGAA ATAAAGCACC CAGCGCTGCC CTGGGCCCCT GCGAGACTGT

6251 GATGGTTCTT TCCACGGGTC AGGCCGAGTC TGAGGCCTGA GTGGCATGAG
6301 ATCTGATATC ATCGATGAAT TCGAGCTCGG TACCCGGGGA TCGATCCAGA
6351 CATGATAAGA TACATTGATG AGTTTGGACA AACCACAACT AGAATGCAGT
6401 GAAAAAAATG CTTTATTTGT GAAATTTGTG ATGCTATTGC TTTATTTGTA
6451 ACCATTATAA GCTGCAATAA ACAAGTTAAC AACAACAATT GCATTCATTT
6501 TATGTTTCAG GTTCAGGGGG AGGTGTGGGA GGTTTTTTAA AGCAAGTAAA
6551 ACCTCTACAA ATGTGGTATG GCTGATTATG ATCTCTAGTC AAGGCACTAT
6601 ACATCAAATA TTCCTTATTA ACCCCTTTAC AAATTAAAAA GCTAAAGGTA
6651 CACAATTTTT GAGCATAGTT ATTAATAGCA GACACTCTAT GCCTGTGTGG
6701 AGTAAGAAAA AACAGTATGT TATGATTATA ACTGTTATGC CTACTTATAA
6751 AGGTTACAGA ATATTTTTCC ATAATTTTCT TGTATAGCAG TGCAGCTTTT
6801 TCCTTTGTGG TGTAAATAGC AAAGCAAGCA AGAGTTCTAT TACTAAACAC
6851 AGCATGACTC AAAAAACTTA GCAATTCTGA AGGAAAGTCC TTGGGGTCTT
6901 CTACCTTTCT CTTCTTTTTT GGAGGAGTAG AATGTTGAGA GTCAGCAGTA
6951 GCCTCATCAT CACTAGATGG CATTTCTTCT GAGCAAAACA GGTTTTCCTC
7001 ATTAAAGGCA TTCCACCACT GCTCCCATTC ATCAGTTCCA TAGGTTGGAA
7051 TCTAAAATAC ACAAACAATT AGAATCAGTA GTTTAACACA TTATACACTT
7101 AAAAATTTTA TATTTACCTT AGAGCTTTAA ATCTCTGTAG GTAGTTTGTC
7151 CAATTATGTC ACACCACAGA AGTAAGGTTC CTTCACAAAG ATCCGGGACC
7201 AAAGCGGCCA TCGTGCCTCC CCACTCCTGC AGTTCGGGGG CATGGATGCG
7251 CGGATAGCCG CTGCTGGTTT CCTGGATGCC GACGGATTTG CACTGCCGGT
7301 AGAACTCCGC GAGGTCGTCC AGCCTCAGGC AGCAGCTGAA CCAACTCGCG
7351 AGGGGATCGA GCCCGGGGTG GGCGAAGAAC TCCAGCATGA GATCCCCGCG
7401 CTGGAGGATC ATCCAGCCGG CGTCCCGGAA AACGATTCCG AAGCCCAACC
7451 TTTCATAGAA GGCGGCGGTG GAATCGAAAT CTCGTGATGG CAGGTTGGGC
7501 GTCGCTTGGT CGGTCATTTC GAACCCCAGA GTCCCGCTCA GAAGAACTCG
7551 TCAAGAAGGC GATAGAAGGC GATGCGCTGC GAATCGGGAG CGGCGATACC
7601 GTAAAGCACG AGGAAGCGGT CAGCCCATTC GCCGCCAAGC TCTTCAGCAA
7651 TATCACGGGT AGCCAACGCT ATGTCCTGAT AGCGGTCCGC CACACCCAGC
7701 CGGCCACAGT CGATGAATCC AGAAAAGCGG CCATTTTCCA CCATGATATT
7751 CGGCAAGCAG GCATCGCCAT GGGTCACGAC GAGATCCTCG CCGTCGGGCA
7801 TGCGCGCCTT GAGCCTGGCG AACAGTTCGG CTGGCGCGAG CCCCTGATGC
7851 TCTTCGTCCA GATCATCCTG ATCGACAAGA CCGGCTTCCA TCCGAGTACG

7901 TGCTCGCTCG ATGCGATGTT TCGCTTGGTG GTCGAATGGG CAGGTAGCCG
7951 GATCAAGCGT ATGCAGCCGC CGCATTGCAT CAGCCATGAT GGATACTTTC
8001 TCGGCAGGAG CAAGGTGAGA TGACAGGAGA TCCTGCCCCG GCACTTCGCC
8051 CAATAGCAGC CAGTCCCTTC CCGCTTCAGT GACAACGTCG AGCACAGCTG
8101 CGCAAGGAAC GCCCGTCGTG GCCAGCCACG ATAGCCGCGC TGCCTCGTCC
8151 TGCAGTTCAT TCAGGGCACC GGACAGGTCG GTCTTGACAA AAAGAACCGG
8201 GCGCCCCTGC GCTGACAGCC GGAACACGGC GGCATCAGAG CAGCCGATTG
8251 TCTGTTGTGC CCAGTCATAG CCGAATAGCC TCTCCACCCA AGCGGCCGGA
8301 GAACCTGCGT GCAATCCATC TTGTTCAATC ATGCGAAACG ATCCTCATCC
8351 TGTCTCTTGA TCAGATCTTG ATCCCCTGCG CCATCAGATC CTTGGCGGCA
8401 AGAAAGCCAT CCAGTTTACT TTGCAGGGCT TCCCAACCTT ACCAGAGGGC
8451 GCCCCAGCTG GCAATTCCGG TTCGCTTGCT GTCCATAAAA CCGCCCAGTC
8501 TAGCTATCGC CATGTAAGCC CACTGCAAGC TACCTGCTTT CTCTTTGCGC
8551 TTGCGTTTTC CCTTGTCCAG ATAGCCCAGT AGCTGACATT CATCCGGGGT
8601 CAGCACCGTT TCTGCGGACT GGCTTTCTAC GTGTTCCGCT TCCTTTAGCA
8651 GCCCTTGCGC CCTGAGTGCT TGCGGCAGCG TGAAGCT

**SEQ ID NO:17**

**Nucleotide sequence of the expression vector HCMV-K HuAb-VL1 hum V1**
**(Complete DNA Sequence of a humanised light chain expression vector comprising SEQ ID NO: 14 (humV1=VLh) from 3964-4284**

1 CTAGCTTTTT GCAAAAGCCT AGGCCTCCAA AAAAGCCTCC TCACTACTTC
51 TGGAATAGCT CAGAGGCCGA GGCGGCCTCG GCCTCTGCAT AAATAAAAAA
101 AATTAGTCAG CCATGGGGCG GAGAATGGGC GGAACTGGGC GGAGTTAGGG
151 GCGGGATGGG CGGAGTTAGG GGCGGGACTA TGGTTGCTGA CTAATTGAGA
201 TGCATGCTTT GCATACTTCT GCCTGCTGGG GAGCCTGGTT GCTGACTAAT
251 TGAGATGCAT GCTTTGCATA CTTCTGCCTG CTGGGGAGCC TGGGGACTTT
301 CCACACCCTA ACTGACACAC ATTCCACAGC TGCCTCGCGC GTTTCGGTGA
351 TGACGGTGAA AACCTCTGAC ACATGCAGCT CCCGGAGACG GTCACAGCTT
401 GTCTGTAAGC GGATGCCGGG AGCAGACAAG CCCGTCAGGG CGCGTCAGCG
451 GGTGTTGGCG GGTGTCGGGG CGCAGCCATG ACCCAGTCAC GTAGCGATAG
501 CGGAGTGTAT ACTGGCTTAA CTATGCGGCA TCAGAGCAGA TTGTACTGAG

551 AGTGCACCAT ATGCGGTGTG AAATACCGCA CAGATGCGTA AGGAGAAAAT
601 ACCGCATCAG GCGCTCTTCC GCTTCCTCGC TCACTGACTC GCTGCGCTCG
651 GTCGTTCGGC TGCGGCGAGC GGTATCAGCT CACTCAAAGG CGGTAATACG
701 GTTATCCACA GAATCAGGGG ATAACGCAGG AAAGAACATG TGAGCAAAAG
751 GCCAGCAAAA GGCCAGGAAC CGTAAAAAGG CCGCGTTGCT GGCGTTTTTC
801 CATAGGCTCC GCCCCCCTGA CGAGCATCAC AAAAATCGAC GCTCAAGTCA
851 GAGGTGGCGA AACCCGACAG GACTATAAAG ATACCAGGCG TTTCCCCCTG
901 GAAGCTCCCT CGTGCGCTCT CCTGTTCCGA CCCTGCCGCT TACCGGATAC
951 CTGTCCGCCT TTCTCCCTTC GGGAAGCGTG GCGCTTTCTC ATAGCTCACG
1001 CTGTAGGTAT CTCAGTTCGG TGTAGGTCGT TCGCTCCAAG CTGGGCTGTG
1051 TGCACGAACC CCCCGTTCAG CCCGACCGCT GCGCCTTATC CGGTAACTAT
1101 CGTCTTGAGT CCAACCCGGT AAGACACGAC TTATCGCCAC TGGCAGCAGC
1151 CACTGGTAAC AGGATTAGCA GAGCGAGGTA TGTAGGCGGT GCTACAGAGT
1201 TCTTGAAGTG GTGGCCTAAC TACGGCTACA CTAGAAGGAC AGTATTTGGT
1251 ATCTGCGCTC TGCTGAAGCC AGTTACCTTC GGAAAAAGAG TTGGTAGCTC
1301 TTGATCCGGC AAACAAACCA CCGCTGGTAG CGGTGGTTTT TTTGTTTGCA
1351 AGCAGCAGAT TACGCGCAGA AAAAAAGGAT CTCAAGAAGA TCCTTTGATC
1401 TTTTCTACGG GGTCTGACGC TCAGTGGAAC GAAAACTCAC GTTAAGGGAT
1451 TTTGGTCATG AGATTATCAA AAAGGATCTT CACCTAGATC CTTTTAAATT
1501 AAAAATGAAG TTTTAAATCA ATCTAAAGTA TATATGAGTA AACTTGGTCT
1551 GACAGTTACC AATGCTTAAT CAGTGAGGCA CCTATCTCAG CGATCTGTCT
1601 ATTTCGTTCA TCCATAGTTG CCTGACTCCC CGTCGTGTAG ATAACTACGA
1651 TACGGGAGGG CTTACCATCT GGCCCCAGTG CTGCAATGAT ACCGCGAGAC
1701 CCACGCTCAC CGGCTCCAGA TTTATCAGCA ATAAACCAGC CAGCCGGAAG
1751 GGCCGAGCGC AGAAGTGGTC CTGCAACTTT ATCCGCCTCC ATCCAGTCTA
1801 TTAATTGTTG CCGGGAAGCT AGAGTAAGTA GTTCGCCAGT TAATAGTTTG
1851 CGCAACGTTG TTGCCATTGC TGCAGGCATC GTGGTGTCAC GCTCGTCGTT
1901 TGGTATGGCT TCATTCAGCT CCGGTTCCCA ACGATCAAGG CGAGTTACAT
1951 GATCCCCCAT GTTGTGCAAA AAAGCGGTTA GCTCCTTCGG TCCTCCGATC
2001 GTTGTCAGAA GTAAGTTGGC CGCAGTGTTA TCACTCATGG TTATGGCAGC
2051 ACTGCATAAT TCTCTTACTG TCATGCCATC CGTAAGATGC TTTTCTGTGA
2101 CTGGTGAGTA CTCAACCAAG TCATTCTGAG AATAGTGTAT GCGGCGACCG
2151 AGTTGCTCTT GCCCGGCGTC AACACGGGAT AATACCGCGC CACATAGCAG

2201 AACTTTAAAA GTGCTCATCA TTGGAAAACG TTCTTCGGGG CGAAAACTCT
2251 CAAGGATCTT ACCGCTGTTG AGATCCAGTT CGATGTAACC CACTCGTGCA
2301 CCCAACTGAT CTTCAGCATC TTTTACTTTC ACCAGCGTTT CTGGGTGAGC
2351 AAAAACAGGA AGGCAAAATG CCGCAAAAAA GGGAATAAGG GCGACACGGA
2401 AATGTTGAAT ACTCATACTC TTCCTTTTTC AATATTATTG AAGCATTTAT
2451 CAGGGTTATT GTCTCATGAG CGGATACATA TTTGAATGTA TTTAGAAAAA
2501 TAAACAAATA GGGGTTCCGC GCACATTTCC CCGAAAAGTG CCACCTGACG
2551 TCTAAGAAAC CATTATTATC ATGACATTAA CCTATAAAAA TAGGCGTATC
2601 ACGAGGCCCT TTCGTCTTCA AGAATTCAGC TTGGCTGCAG TGAATAATAA
2651 AATGTGTGTT TGTCCGAAAT ACGCGTTTTG AGATTTCTGT CGCCGACTAA
2701 ATTCATGTCG CGCGATAGTG GTGTTTATCG CCGATAGAGA TGGCGATATT
2751 GGAAAAATCG ATATTTGAAA ATATGGCATA TTGAAAATGT CGCCGATGTG
2801 AGTTTCTGTG TAACTGATAT CGCCATTTTT CCAAAAGTGA TTTTTGGGCA
2851 TACGCGATAT CTGGCGATAG CGCTTATATC GTTTACGGGG GATGGCGATA
2901 GACGACTTTG GTGACTTGGG CGATTCTGTG TGTCGCAAAT ATCGCAGTTT
2951 CGATATAGGT GACAGACGAT ATGAGGCTAT ATCGCCGATA GAGGCGACAT
3001 CAAGCTGGCA CATGGCCAAT GCATATCGAT CTATACATTG AATCAATATT
3051 GGCCATTAGC CATATTATTC ATTGGTTATA TAGCATAAAT CAATATTGGC
3101 TATTGGCCAT TGCATACGTT GTATCCATAT CATAATATGT ACATTTATAT
3151 TGGCTCATGT CCAACATTAC CGCCATGTTG ACATTGATTA TTGACTAGTT
3201 ATTAATAGTA ATCAATTACG GGGTCATTAG TTCATAGCCC ATATATGGAG
3251 TTCCGCGTTA CATAACTTAC GGTAAATGGC CCGCCTGGCT GACCGCCCAA
3301 CGACCCCCGC CCATTGACGT CAATAATGAC GTATGTTCCC ATAGTAACGC
3351 CAATAGGGAC TTTCCATTGA CGTCAATGGG TGGAGTATTT ACGGTAAACT
3401 GCCCACTTGG CAGTACATCA AGTGTATCAT ATGCCAAGTA CGCCCCCTAT
3451 TGACGTCAAT GACGGTAAAT GGCCCGCCTG GCATTATGCC CAGTACATGA
3501 CCTTATGGGA CTTTCCTACT TGGCAGTACA TCTACGTATT AGTCATCGCT
3551 ATTACCATGG TGATGCGGTT TTGGCAGTAC ATCAATGGGC GTGGATAGCG
3601 GTTTGACTCA CGGGGATTTC CAAGTCTCCA CCCCATTGAC GTCAATGGGA
3651 GTTTGTTTTG GCACCAAAAT CAACGGGACT TTCCAAAATG TCGTAACAAC
3701 TCCGCCCCAT TGACGCAAAT GGGCGGTAGG CGTGTACGGT GGGAGGTCTA
3751 TATAAGCAGA GCTCGTTTAG TGAACCGTCA GATCGCCTGG AGACGCCATC
3801 CACGCTGTTT TGACCTCCAT AGAAGACACC GGGACCGATC CAGCCTCCGC

3851 AAGCTTGATA TCGAATTCCT GCAGCCCGGG GGATCCGCCC GCTTGCCGCC
3901 ACCATGGAGA CCCCCGCCCA GCTGCTGTTC CTGCTGCTGC TGTGGCTGCC
3951 CGACACCACC GGCGACATTC TGCTGACCCA GTCTCCAGCC ACCCTGTCTC
4001 TGAGTCCAGG AGAAAGAGCC ACTCTCTCCT GCAGGGCCAG TCAGAACATT
4051 GGCACAAGCA TACAGTGGTA TCAACAAAAA CCAGGTCAGG CTCCAAGGCT
4101 TCTCATAAGG TCTTCTTCTG AGTCTATCTC TGGGATCCCT TCCAGGTTTA
4151 GTGGCAGTGG ATCAGGGACA GATTTTACTC TTACCATCAG CAGTCTGGAG
4201 CCTGAAGATT TTGCAGTGTA TTACTGTCAA CAAAGTAATA CCTGGCCATT
4251 CACGTTCGGC CAGGGGACCA AGCTGGAGAT CAAACGTGAG TATTCTAGAA
4301 AGATCCTAGA ATTCTAAACT CTGAGGGGGT CGGATGACGT GGCCATTCTT
4351 TGCCTAAAGC ATTGAGTTTA CTGCAAGGTC AGAAAAGCAT GCAAAGCCCT
4401 CAGAATGGCT GCAAAGAGCT CCAACAAAAC AATTTAGAAC TTTATTAAGG
4451 AATAGGGGGA AGCTAGGAAG AAACTCAAAA CATCAAGATT TTAAATACGC
4501 TTCTTGGTCT CCTTGCTATA ATTATCTGGG ATAAGCATGC TGTTTTCTGT
4551 CTGTCCCTAA CATGCCCTGT GATTATCCGC AAACAACACA CCCAAGGGCA
4601 GAACTTTGTT ACTTAAACAC CATCCTGTTT GCTTCTTTCC TCAGGAACTG
4651 TGGCTGCACC ATCTGTCTTC ATCTTCCCGC CATCTGATGA GCAGTTGAAA
4701 TCTGGAACTG CCTCTGTTGT GTGCCTGCTG AATAACTTCT ATCCCAGAGA
4751 GGCCAAAGTA CAGTGGAAGG TGGATAACGC CCTCCAATCG GGTAACTCCC
4801 AGGAGAGTGT CACAGAGCAG GACAGCAAGG ACAGCACCTA CAGCCTCAGC
4851 AGCACCCTGA CGCTGAGCAA AGCAGACTAC GAGAAACACA AAGTCTACGC
4901 CTGCGAAGTC ACCCATCAGG GCCTGAGCTC GCCCGTCACA AAGAGCTTCA
4951 ACAGGGGAGA GTGTTAGAGG GAGAAGTGCC CCCACCTGCT CCTCAGTTCC
5001 AGCCTGACCC CCTCCCATCC TTTGGCCTCT GACCCTTTTT CCACAGGGGA
5051 CCTACCCCTA TTGCGGTCCT CCAGCTCATC TTTCACCTCA CCCCCCTCCT
5101 CCTCCTTGGC TTTAATTATG CTAATGTTGG AGGAGAATGA ATAAATAAAG
5151 TGAATCTTTG CACCTGTGGT TTCTCTCTTT CCTCATTTAA TAATTATTAT
5201 CTGTTGTTTA CCAACTACTC AATTTCTCTT ATAAGGGACT AAATATGTAG
5251 TCATCCTAAG GCGCATAACC ATTTATAAAA ATCATCCTTC ATTCTATTTT
5301 ACCCTATCAT CCTCTGCAAG ACAGTCCTCC CTCAAACCCA CAAGCCTTCT
5351 GTCCTCACAG TCCCCTGGGC CATGGTAGGA GAGACTTGCT TCCTTGTTTT
5401 CCCCTCCTCA GCAAGCCCTC ATAGTCCTTT TTAAGGGTGA CAGGTCTTAC
5451 AGTCATATAT CCTTTGATTC AATTCCCTGA GAATCAACCA AAGCAAATTT

5501 TTCAAAAGAA GAAACCTGCT ATAAAGAGAA TCATTCATTG CAACATGATA
5551 TAAAATAACA ACACAATAAA AGCAATTAAA TAAACAAACA ATAGGGAAAT
5601 GTTTAAGTTC ATCATGGTAC TTAGACTTAA TGGAATGTCA TGCCTTATTT
5651 ACATTTTTAA ACAGGTACTG AGGGACTCCT GTCTGCCAAG GGCCGTATTG
5701 AGTACTTTCC ACAACCTAAT TTAATCCACA CTATACTGTG AGATTAAAAA
5751 CATTCATTAA AATGTTGCAA AGGTTCTATA AAGCTGAGAG ACAAATATAT
5801 TCTATAACTC AGCAATCCCA CTTCTAGATG ACTGAGTGTC CCCACCCACC
5851 AAAAAACTAT GCAAGAATGT TCAAAGCAGC TTTATTTACA AAAGCCAAAA
5901 ATTGGAAATA GCCCGATTGT CCAACAATAG AATGAGTTAT TAAACTGTGG
5951 TATGTTTATA CATTAGAATA CCCAATGAGG AGAATTAACA AGCTACAACT
6001 ATACCTACTC ACACAGATGA ATCTCATAAA AATAATGTTA CATAAGAGAA
6051 ACTCAATGCA AAAGATATGT TCTGTATGTT TTCATCCATA TAAAGTTCAA
6101 AACCAGGTAA AAATAAAGTT AGAAATTTGG ATGGAAATTA CTCTTAGCTG
6151 GGGGTGGGCG AGTTAGTGCC TGGGAGAAGA CAAGAAGGGG CTTCTGGGGT
6201 CTTGGTAATG TTCTGTTCCT CGTGTGGGGT TGTGCAGTTA TGATCTGTGC
6251 ACTGTTCTGT ATACACATTA TGCTTCAAAA TAACTTCACA TAAAGAACAT
6301 CTTATACCCA GTTAATAGAT AGAAGAGGAA TAAGTAATAG GTCAAGACCA
6351 CGCAGCTGGT AAGTGGGGGG GCCTGGGATC AAATAGCTAC CTGCCTAATC
6401 CTGCCCTCTT GAGCCCTGAA TGAGTCTGCC TTCCAGGGCT CAAGGTGCTC
6451 AACAAAACAA CAGGCCTGCT ATTTTCCTGG CATCTGTGCC CTGTTTGGCT
6501 AGCTAGGAGC ACACATACAT AGAAATTAAA TGAAACAGAC CTTCAGCAAG
6551 GGGACAGAGG ACAGAATTAA CCTTGCCCAG ACACTGGAAA CCCATGTATG
6601 AACACTCACA TGTTTGGGAA GGGGGAAGGG CACATGTAAA TGAGGACTCT
6651 TCCTCATTCT ATGGGGCACT CTGGCCCTGC CCCTCTCAGC TACTCATCCA
6701 TCCAACACAC CTTTCTAAGT ACCTCTCTCT GCCTACACTC TGAAGGGGTT
6751 CAGGAGTAAC TAACACAGCA TCCCTTCCCT CAAATGACTG ACAATCCCTT
6801 TGTCCTGCTT TGTTTTTCTT TCCAGTCAGT ACTGGGAAAG TGGGGAAGGA
6851 CAGTCATGGA GAAACTACAT AAGGAAGCAC CTTGCCCTTC TGCCTCTTGA
6901 GAATGTTGAT GAGTATCAAA TCTTTCAAAC TTTGGAGGTT TGAGTAGGGG
6951 TGAGACTCAG TAATGTCCCT TCCAATGACA TGAACTTGCT CACTCATCCC
7001 TGGGGGCCAA ATTGAACAAT CAAAGGCAGG CATAATCCAG CTATGAATTC
7051 TAGGATCGAT CCAGACATGA TAAGATACAT TGATGAGTTT GGACAAACCA
7101 CAACTAGAAT GCAGTGAAAA AAATGCTTTA TTTGTGAAAT TTGTGATGCT

7151 ATTGCTTTAT TTGTAACCAT TATAAGCTGC AATAAACAAG TTAACAACAA
7201 CAATTGCATT CATTTTATGT TTCAGGTTCA GGGGGAGGTG TGGGAGGTTT
7251 TTTAAAGCAA GTAAAACCTC TACAAATGTG GTATGGCTGA TTATGATCTC
7301 TAGTCAAGGC ACTATACATC AAATATTCCT TATTAACCCC TTTACAAATT
7351 AAAAAGCTAA AGGTACACAA TTTTTGAGCA TAGTTATTAA TAGCAGACAC
7401 TCTATGCCTG TGTGGAGTAA GAAAAAACAG TATGTTATGA TTATAACTGT
7451 TATGCCTACT TATAAAGGTT ACAGAATATT TTTCCATAAT TTTCTTGTAT
7501 AGCAGTGCAG CTTTTTCCTT TGTGGTGTAA ATAGCAAAGC AAGCAAGAGT
7551 TCTATTACTA AACACAGCAT GACTCAAAAA ACTTAGCAAT TCTGAAGGAA
7601 AGTCCTTGGG GTCTTCTACC TTTCTCTTCT TTTTTGGAGG AGTAGAATGT
7651 TGAGAGTCAG CAGTAGCCTC ATCATCACTA GATGGCATTT CTTCTGAGCA
7701 AAACAGGTTT TCCTCATTAA AGGCATTCCA CCACTGCTCC CATTCATCAG
7751 TTCCATAGGT TGGAATCTAA AATACACAAA CAATTAGAAT CAGTAGTTTA
7801 ACACATTATA CACTTAAAAA TTTTATATTT ACCTTAGAGC TTTAAATCTC
7851 TGTAGGTAGT TTGTCCAATT ATGTCACACC ACAGAAGTAA GGTTCCTTCA
7901 CAAAGATCCG GGACCAAAGC GGCCATCGTG CCTCCCCACT CCTGCAGTTC
7951 GGGGGCATGG ATGCGCGGAT AGCCGCTGCT GGTTTCCTGG ATGCCGACGG
8001 ATTTGCACTG CCGGTAGAAC TCCGCGAGGT CGTCCAGCCT CAGGCAGCAG
8051 CTGAACCAAC TCGCGAGGGG ATCGAGCCCG GGGTGGGCGA AGAACTCCAG
8101 CATGAGATCC CCGCGCTGGA GGATCATCCA GCCGGCGTCC CGGAAAACGA
8151 TTCCGAAGCC CAACCTTTCA TAGAAGGCGG CGGTGGAATC GAAATCTCGT
8201 GATGGCAGGT TGGGCGTCGC TTGGTCGGTC ATTTCGAACC CCAGAGTCCC
8251 GCTCAGAAGA ACTCGTCAAG AAGGCGATAG AAGGCGATGC GCTGCGAATC
8301 GGGAGCGGCG ATACCGTAAA GCACGAGGAA GCGGTCAGCC CATTCGCCGC
8351 CAAGCTCTTC AGCAATATCA CGGGTAGCCA ACGCTATGTC CTGATAGCGG
8401 TCCGCCACAC CCAGCCGGCC ACAGTCGATG AATCCAGAAA AGCGGCCATT
8451 TTCCACCATG ATATTCGGCA AGCAGGCATC GCCATGGGTC ACGACGAGAT
8501 CCTCGCCGTC GGGCATGCGC GCCTTGAGCC TGGCGAACAG TTCGGCTGGC
8551 GCGAGCCCCT GATGCTCTTC GTCCAGATCA TCCTGATCGA CAAGACCGGC
8601 TTCCATCCGA GTACGTGCTC GCTCGATGCG ATGTTTCGCT TGGTGGTCGA
8651 ATGGGCAGGT AGCCGGATCA AGCGTATGCA GCCGCCGCAT TGCATCAGCC
8701 ATGATGGATA CTTTCTCGGC AGGAGCAAGG TGAGATGACA GGAGATCCTG
8751 CCCCGGCACT TCGCCCAATA GCAGCCAGTC CCTTCCCGCT TCAGTGACAA

8801 CGTCGAGCAC AGCTGCGCAA GGAACGCCCG TCGTGGCCAG CCACGATAGC
8851 CGCGCTGCCT CGTCCTGCAG TTCATTCAGG GCACCGGACA GGTCGGTCTT
8901 GACAAAAAGA ACCGGGCGCC CCTGCGCTGA CAGCCGGAAC ACGGCGGCAT
8951 CAGAGCAGCC GATTGTCTGT TGTGCCCAGT CATAGCCGAA TAGCCTCTCC
9001 ACCCAAGCGG CCGGAGAACC TGCGTGCAAT CCATCTTGTT CAATCATGCG
9051 AAACGATCCT CATCCTGTCT CTTGATCAGA TCTTGATCCC CTGCGCCATC
9101 AGATCCTTGG CGGCAAGAAA GCCATCCAGT TTACTTTGCA GGGCTTCCCA
9151 ACCTTACCAG AGGGCGCCCC AGCTGGCAAT TCCGGTTCGC TTGCTGTCCA
9201 TAAAACCGCC CAGTCTAGCT ATCGCCATGT AAGCCCACTG CAAGCTACCT
9251 GCTTTCTCTT TGCGCTTGCG TTTTCCCTTG TCCAGATAGC CCAGTAGCTG
9301 ACATTCATCC GGGGTCAGCA CCGTTTCTGC GGACTGGCTT TCTACGTGTT
9351 CCGCTTCCTT TAGCAGCCCT TGCGCCCTGA GTGCTTGCGG CAGCGTGAAG

SEQ ID NO:18

**Nucleotide sequence of the expression vector HCMV-K HuAb-VL1 hum V2 (Complete DNA Sequence of a humanised light chain expression vector comprising SEQ ID NO: 13 (humV2=VLm) from 3926-4246)**

1 CTAGCTTTTT GCAAAAGCCT AGGCCTCCAA AAAAGCCTCC TCACTACTTC
51 TGGAATAGCT CAGAGGCCGA GGCGGCCTCG GCCTCTGCAT AAATAAAAAA
101 AATTAGTCAG CCATGGGGCG GAGAATGGGC GGAACTGGGC GGAGTTAGGG
151 GCGGGATGGG CGGAGTTAGG GGCGGGACTA TGGTTGCTGA CTAATTGAGA
201 TGCATGCTTT GCATACTTCT GCCTGCTGGG GAGCCTGGTT GCTGACTAAT
251 TGAGATGCAT GCTTTGCATA CTTCTGCCTG CTGGGGAGCC TGGGGACTTT
301 CCACACCCTA ACTGACACAC ATTCCACAGC TGCCTCGCGC GTTTCGGTGA
351 TGACGGTGAA AACCTCTGAC ACATGCAGCT CCCGGAGACG GTCACAGCTT
401 GTCTGTAAGC GGATGCCGGG AGCAGACAAG CCCGTCAGGG CGCGTCAGCG
451 GGTGTTGGCG GGTGTCGGGG CGCAGCCATG ACCCAGTCAC GTAGCGATAG
501 CGGAGTGTAT ACTGGCTTAA CTATGCGGCA TCAGAGCAGA TTGTACTGAG
551 AGTGCACCAT ATGCGGTGTG AAATACCGCA CAGATGCGTA AGGAGAAAAT
601 ACCGCATCAG GCGCTCTTCC GCTTCCTCGC TCACTGACTC GCTGCGCTCG
651 GTCGTTCGGC TGCGGCGAGC GGTATCAGCT CACTCAAAGG CGGTAATACG
701 GTTATCCACA GAATCAGGGG ATAACGCAGG AAAGAACATG TGAGCAAAAG

751 GCCAGCAAAA GGCCAGGAAC CGTAAAAAGG CCGCGTTGCT GGCGTTTTTC
801 CATAGGCTCC GCCCCCCTGA CGAGCATCAC AAAAATCGAC GCTCAAGTCA
851 GAGGTGGCGA AACCCGACAG GACTATAAAG ATACCAGGCG TTTCCCCCTG
901 GAAGCTCCCT CGTGCGCTCT CCTGTTCCGA CCCTGCCGCT TACCGGATAC
951 CTGTCCGCCT TTCTCCCTTC GGGAAGCGTG GCGCTTTCTC ATAGCTCACG
1001 CTGTAGGTAT CTCAGTTCGG TGTAGGTCGT TCGCTCCAAG CTGGGCTGTG
1051 TGCACGAACC CCCCGTTCAG CCCGACCGCT GCGCCTTATC CGGTAACTAT
1101 CGTCTTGAGT CCAACCCGGT AAGACACGAC TTATCGCCAC TGGCAGCAGC
1151 CACTGGTAAC AGGATTAGCA GAGCGAGGTA TGTAGGCGGT GCTACAGAGT
1201 TCTTGAAGTG GTGGCCTAAC TACGGCTACA CTAGAAGGAC AGTATTTGGT
1251 ATCTGCGCTC TGCTGAAGCC AGTTACCTTC GGAAAAAGAG TTGGTAGCTC
1301 TTGATCCGGC AAACAAACCA CCGCTGGTAG CGGTGGTTTT TTTGTTTGCA
1351 AGCAGCAGAT TACGCGCAGA AAAAAAGGAT CTCAAGAAGA TCCTTTGATC
1401 TTTTCTACGG GGTCTGACGC TCAGTGGAAC GAAAACTCAC GTTAAGGGAT
1451 TTTGGTCATG AGATTATCAA AAAGGATCTT CACCTAGATC CTTTTAAATT
1501 AAAAATGAAG TTTTAAATCA ATCTAAAGTA TATATGAGTA AACTTGGTCT
1551 GACAGTTACC AATGCTTAAT CAGTGAGGCA CCTATCTCAG CGATCTGTCT
1601 ATTTCGTTCA TCCATAGTTG CCTGACTCCC CGTCGTGTAG ATAACTACGA
1651 TACGGGAGGG CTTACCATCT GGCCCCAGTG CTGCAATGAT ACCGCGAGAC
1701 CCACGCTCAC CGGCTCCAGA TTTATCAGCA ATAAACCAGC CAGCCGGAAG
1751 GGCCGAGCGC AGAAGTGGTC CTGCAACTTT ATCCGCCTCC ATCCAGTCTA
1801 TTAATTGTTG CCGGGAAGCT AGAGTAAGTA GTTCGCCAGT TAATAGTTTG
1851 CGCAACGTTG TTGCCATTGC TGCAGGCATC GTGGTGTCAC GCTCGTCGTT
1901 TGGTATGGCT TCATTCAGCT CCGGTTCCCA ACGATCAAGG CGAGTTACAT
1951 GATCCCCCAT GTTGTGCAAA AAAGCGGTTA GCTCCTTCGG TCCTCCGATC
2001 GTTGTCAGAA GTAAGTTGGC CGCAGTGTTA TCACTCATGG TTATGGCAGC
2051 ACTGCATAAT TCTCTTACTG TCATGCCATC CGTAAGATGC TTTTCTGTGA
2101 CTGGTGAGTA CTCAACCAAG TCATTCTGAG AATAGTGTAT GCGGCGACCG
2151 AGTTGCTCTT GCCCGGCGTC AACACGGGAT AATACCGCGC CACATAGCAG
2201 AACTTTAAAA GTGCTCATCA TTGGAAAACG TTCTTCGGGG CGAAAACTCT
2251 CAAGGATCTT ACCGCTGTTG AGATCCAGTT CGATGTAACC CACTCGTGCA
2301 CCCAACTGAT CTTCAGCATC TTTTACTTTC ACCAGCGTTT CTGGGTGAGC
2351 AAAAACAGGA AGGCAAAATG CCGCAAAAAA GGGAATAAGG GCGACACGGA

```
2401  AATGTTGAAT ACTCATACTC TTCCTTTTTC AATATTATTG AAGCATTTAT
2451  CAGGGTTATT GTCTCATGAG CGGATACATA TTTGAATGTA TTTAGAAAAA
2501  TAAACAAATA GGGGTTCCGC GCACATTTCC CCGAAAAGTG CCACCTGACG
2551  TCTAAGAAAC CATTATTATC ATGACATTAA CCTATAAAAA TAGGCGTATC
2601  ACGAGGCCCT TTCGTCTTCA AGAATTCAGC TTGGCTGCAG TGAATAATAA
2651  AATGTGTGTT TGTCCGAAAT ACGCGTTTTG AGATTTCTGT CGCCGACTAA
2701  ATTCATGTCG CGCGATAGTG GTGTTTATCG CCGATAGAGA TGGCGATATT
2751  GGAAAAATCG ATATTTGAAA ATATGGCATA TTGAAAATGT CGCCGATGTG
2801  AGTTTCTGTG TAACTGATAT CGCCATTTTT CCAAAAGTGA TTTTTGGGCA
2851  TACGCGATAT CTGGCGATAG CGCTTATATC GTTTACGGGG GATGGCGATA
2901  GACGACTTTG GTGACTTGGG CGATTCTGTG TGTCGCAAAT ATCGCAGTTT
2951  CGATATAGGT GACAGACGAT ATGAGGCTAT ATCGCCGATA GAGGCGACAT
3001  CAAGCTGGCA CATGGCCAAT GCATATCGAT CTATACATTG AATCAATATT
3051  GGCCATTAGC CATATTATTC ATTGGTTATA TAGCATAAAT CAATATTGGC
3101  TATTGGCCAT TGCATACGTT GTATCCATAT CATAATATGT ACATTTATAT
3151  TGGCTCATGT CCAACATTAC CGCCATGTTG ACATTGATTA TTGACTAGTT
3201  ATTAATAGTA ATCAATTACG GGGTCATTAG TTCATAGCCC ATATATGGAG
3251  TTCCGCGTTA CATAACTTAC GGTAAATGGC CCGCCTGGCT GACCGCCCAA
3301  CGACCCCCGC CCATTGACGT CAATAATGAC GTATGTTCCC ATAGTAACGC
3351  CAATAGGGAC TTTCCATTGA CGTCAATGGG TGGAGTATTT ACGGTAAACT
3401  GCCCACTTGG CAGTACATCA AGTGTATCAT ATGCCAAGTA CGCCCCCTAT
3451  TGACGTCAAT GACGGTAAAT GGCCCGCCTG GCATTATGCC CAGTACATGA
3501  CCTTATGGGA CTTTCCTACT TGGCAGTACA TCTACGTATT AGTCATCGCT
3551  ATTACCATGG TGATGCGGTT TTGGCAGTAC ATCAATGGGC GTGGATAGCG
3601  GTTTGACTCA CGGGGATTTC CAAGTCTCCA CCCCATTGAC GTCAATGGGA
3651  GTTTGTTTTG GCACCAAAAT CAACGGGACT TTCCAAAATG TCGTAACAAC
3701  TCCGCCCCAT TGACGCAAAT GGGCGGTAGG CGTGTACGGT GGGAGGTCTA
3751  TATAAGCAGA GCTCGTTTAG TGAACCGTCA GATCGCCTGG AGACGCCATC
3801  CACGCTGTTT TGACCTCCAT AGAAGACACC GGGACCGATC CAGCCTCCGC
3851  AAGCTTGCCG CCACCATGGA GACCCCCGCC CAGCTGCTGT TCCTGCTGCT
3901  GCTGTGGCTG CCCGACACCA CCGGCGACAT TCTGCTGACC CAGTCTCCAG
3951  CCACCCTGTC TCTGAGTCCA GGAGAAAGAG CCACTTTCTC CTGCAGGGCC
4001  AGTCAGAACA TTGGCACAAG CATACAGTGG TATCAACAAA AAACAAATGG
```

4051 TGCTCCAAGG CTTCTCATAA GGTCTTCTTC TGAGTCTATC TCTGGGATCC
4101 CTTCCAGGTT TAGTGGCAGT GGATCAGGGA CAGATTTTAC TCTTACCATC
4151 AGCAGTCTGG AGCCTGAAGA TTTTGCAGTG TATTACTGTC AACAAAGTAA
4201 TACCTGGCCA TTCACGTTCG GCCAGGGGAC CAAGCTGGAG ATCAAACGTG
4251 AGTATTCTAG AAAGATCCTA GAATTCTAAA CTCTGAGGGG GTCGGATGAC
4301 GTGGCCATTC TTTGCCTAAA GCATTGAGTT TACTGCAAGG TCAGAAAAGC
4351 ATGCAAAGCC CTCAGAATGG CTGCAAAGAG CTCCAACAAA ACAATTTAGA
4401 ACTTTATTAA GGAATAGGGG GAAGCTAGGA AGAAACTCAA AACATCAAGA
4451 TTTTAAATAC GCTTCTTGGT CTCCTTGCTA TAATTATCTG GGATAAGCAT
4501 GCTGTTTTCT GTCTGTCCCT AACATGCCCT GTGATTATCC GCAAACAACA
4551 CACCCAAGGG CAGAACTTTG TTACTTAAAC ACCATCCTGT TTGCTTCTTT
4601 CCTCAGGAAC TGTGGCTGCA CCATCTGTCT TCATCTTCCC GCCATCTGAT
4651 GAGCAGTTGA AATCTGGAAC TGCCTCTGTT GTGTGCCTGC TGAATAACTT
4701 CTATCCCAGA GAGGCCAAAG TACAGTGGAA GGTGGATAAC GCCCTCCAAT
4751 CGGGTAACTC CCAGGAGAGT GTCACAGAGC AGGACAGCAA GGACAGCACC
4801 TACAGCCTCA GCAGCACCCT GACGCTGAGC AAAGCAGACT ACGAGAAACA
4851 CAAAGTCTAC GCCTGCGAAG TCACCCATCA GGGCCTGAGC TCGCCCGTCA
4901 CAAAGAGCTT CAACAGGGGA GAGTGTTAGA GGGAGAAGTG CCCCCACCTG
4951 CTCCTCAGTT CCAGCCTGAC CCCCTCCCAT CCTTTGGCCT CTGACCCTTT
5001 TTCCACAGGG GACCTACCCC TATTGCGGTC CTCCAGCTCA TCTTTCACCT
5051 CACCCCCCTC CTCCTCCTTG GCTTTAATTA TGCTAATGTT GGAGGAGAAT
5101 GAATAAATAA AGTGAATCTT TGCACCTGTG GTTTCTCTCT TTCCTCATTT
5151 AATAATTATT ATCTGTTGTT TACCAACTAC TCAATTTCTC TTATAAGGGA
5201 CTAAATATGT AGTCATCCTA AGGCGCATAA CCATTTATAA AAATCATCCT
5251 TCATTCTATT TTACCCTATC ATCCTCTGCA AGACAGTCCT CCCTCAAACC
5301 CACAAGCCTT CTGTCCTCAC AGTCCCCTGG GCCATGGTAG GAGAGACTTG
5351 CTTCCTTGTT TTCCCCTCCT CAGCAAGCCC TCATAGTCCT TTTTAAGGGT
5401 GACAGGTCTT ACAGTCATAT ATCCTTTGAT TCAATTCCCT GAGAATCAAC
5451 CAAAGCAAAT TTTTCAAAAG AAGAAACCTG CTATAAAGAG AATCATTCAT
5501 TGCAACATGA TATAAAATAA CAACACAATA AAAGCAATTA AATAAACAAA
5551 CAATAGGGAA ATGTTTAAGT TCATCATGGT ACTTAGACTT AATGGAATGT
5601 CATGCCTTAT TTACATTTTT AAACAGGTAC TGAGGGACTC CTGTCTGCCA
5651 AGGGCCGTAT TGAGTACTTT CCACAACCTA ATTTAATCCA CACTATACTG

5701 TGAGATTAAA AACATTCATT AAAATGTTGC AAAGGTTCTA TAAAGCTGAG
5751 AGACAAATAT ATTCTATAAC TCAGCAATCC CACTTCTAGA TGACTGAGTG
5801 TCCCCACCCA CCAAAAAACT ATGCAAGAAT GTTCAAAGCA GCTTTATTTA
5851 CAAAAGCCAA AAATTGGAAA TAGCCCGATT GTCCAACAAT AGAATGAGTT
5901 ATTAAACTGT GGTATGTTTA TACATTAGAA TACCCAATGA GGAGAATTAA
5951 CAAGCTACAA CTATACCTAC TCACACAGAT GAATCTCATA AAAATAATGT
6001 TACATAAGAG AAACTCAATG CAAAAGATAT GTTCTGTATG TTTTCATCCA
6051 TATAAAGTTC AAAACCAGGT AAAAATAAAG TTAGAAATTT GGATGGAAAT
6101 TACTCTTAGC TGGGGGTGGG CGAGTTAGTG CCTGGGAGAA GACAAGAAGG
6151 GGCTTCTGGG GTCTTGGTAA TGTTCTGTTC CTCGTGTGGG GTTGTGCAGT
6201 TATGATCTGT GCACTGTTCT GTATACACAT TATGCTTCAA AATAACTTCA
6251 CATAAAGAAC ATCTTATACC CAGTTAATAG ATAGAAGAGG AATAAGTAAT
6301 AGGTCAAGAC CACGCAGCTG GTAAGTGGGG GGGCCTGGGA TCAAATAGCT
6351 ACCTGCCTAA TCCTGCCCTC TTGAGCCCTG AATGAGTCTG CCTTCCAGGG
6401 CTCAAGGTGC TCAACAAAAC AACAGGCCTG CTATTTTCCT GGCATCTGTG
6451 CCCTGTTTGG CTAGCTAGGA GCACACATAC ATAGAAATTA AATGAAACAG
6501 ACCTTCAGCA AGGGGACAGA GGACAGAATT AACCTTGCCC AGACACTGGA
6551 AACCCATGTA TGAACACTCA CATGTTTGGG AAGGGGGAAG GGCACATGTA
6601 AATGAGGACT CTTCCTCATT CTATGGGGCA CTCTGGCCCT GCCCCTCTCA
6651 GCTACTCATC CATCCAACAC ACCTTTCTAA GTACCTCTCT CTGCCTACAC
6701 TCTGAAGGGG TTCAGGAGTA ACTAACACAG CATCCCTTCC CTCAAATGAC
6751 TGACAATCCC TTTGTCCTGC TTTGTTTTTC TTTCCAGTCA GTACTGGGAA
6801 AGTGGGGAAG GACAGTCATG GAGAAACTAC ATAAGGAAGC ACCTTGCCCT
6851 TCTGCCTCTT GAGAATGTTG ATGAGTATCA AATCTTTCAA ACTTTGGAGG
6901 TTTGAGTAGG GGTGAGACTC AGTAATGTCC CTTCCAATGA CATGAACTTG
6951 CTCACTCATC CCTGGGGGCC AAATTGAACA ATCAAAGGCA GGCATAATCC
7001 AGCTATGAAT TCTAGGATCG ATCCAGACAT GATAAGATAC ATTGATGAGT
7051 TTGGACAAAC CACAACTAGA ATGCAGTGAA AAAAATGCTT TATTTGTGAA
7101 ATTTGTGATG CTATTGCTTT ATTTGTAACC ATTATAAGCT GCAATAAACA
7151 AGTTAACAAC AACAATTGCA TTCATTTTAT GTTTCAGGTT CAGGGGGAGG
7201 TGTGGGAGGT TTTTTAAAGC AAGTAAAACC TCTACAAATG TGGTATGGCT
7251 GATTATGATC TCTAGTCAAG GCACTATACA TCAAATATTC CTTATTAACC
7301 CCTTTACAAA TTAAAAAGCT AAAGGTACAC AATTTTTGAG CATAGTTATT

7351 AATAGCAGAC ACTCTATGCC TGTGTGGAGT AAGAAAAAAC AGTATGTTAT
7401 GATTATAACT GTTATGCCTA CTTATAAAGG TTACAGAATA TTTTTCCATA
7451 ATTTTCTTGT ATAGCAGTGC AGCTTTTTCC TTTGTGGTGT AAATAGCAAA
7501 GCAAGCAAGA GTTCTATTAC TAAACACAGC ATGACTCAAA AAACTTAGCA
7551 ATTCTGAAGG AAAGTCCTTG GGGTCTTCTA CCTTTCTCTT CTTTTTTGGA
7601 GGAGTAGAAT GTTGAGAGTC AGCAGTAGCC TCATCATCAC TAGATGGCAT
7651 TTCTTCTGAG CAAAACAGGT TTTCCTCATT AAAGGCATTC CACCACTGCT
7701 CCCATTCATC AGTTCCATAG GTTGGAATCT AAAATACACA AACAATTAGA
7751 ATCAGTAGTT TAACACATTA TACACTTAAA AATTTTATAT TTACCTTAGA
7801 GCTTTAAATC TCTGTAGGTA GTTTGTCCAA TTATGTCACA CCACAGAAGT
7851 AAGGTTCCTT CACAAAGATC CGGGACCAAA GCGGCCATCG TGCCTCCCCA
7901 CTCCTGCAGT TCGGGGGCAT GGATGCGCGG ATAGCCGCTG CTGGTTTCCT
7951 GGATGCCGAC GGATTTGCAC TGCCGGTAGA ACTCCGCGAG GTCGTCCAGC
8001 CTCAGGCAGC AGCTGAACCA ACTCGCGAGG GGATCGAGCC CGGGGTGGGC
8051 GAAGAACTCC AGCATGAGAT CCCCGCGCTG GAGGATCATC CAGCCGGCGT
8101 CCCGGAAAAC GATTCCGAAG CCCAACCTTT CATAGAAGGC GGCGGTGGAA
8151 TCGAAATCTC GTGATGGCAG GTTGGGCGTC GCTTGGTCGG TCATTTCGAA
8201 CCCCAGAGTC CCGCTCAGAA GAACTCGTCA AGAAGGCGAT AGAAGGCGAT
8251 GCGCTGCGAA TCGGGAGCGG CGATACCGTA AAGCACGAGG AAGCGGTCAG
8301 CCCATTCGCC GCCAAGCTCT TCAGCAATAT CACGGGTAGC CAACGCTATG
8351 TCCTGATAGC GGTCCGCCAC ACCCAGCCGG CCACAGTCGA TGAATCCAGA
8401 AAAGCGGCCA TTTTCCACCA TGATATTCGG CAAGCAGGCA TCGCCATGGG
8451 TCACGACGAG ATCCTCGCCG TCGGGCATGC GCGCCTTGAG CCTGGCGAAC
8501 AGTTCGGCTG GCGCGAGCCC CTGATGCTCT TCGTCCAGAT CATCCTGATC
8551 GACAAGACCG GCTTCCATCC GAGTACGTGC TCGCTCGATG CGATGTTTCG
8601 CTTGGTGGTC GAATGGGCAG GTAGCCGGAT CAAGCGTATG CAGCCGCCGC
8651 ATTGCATCAG CCATGATGGA TACTTTCTCG GCAGGAGCAA GGTGAGATGA
8701 CAGGAGATCC TGCCCCGGCA CTTCGCCCAA TAGCAGCCAG TCCCTTCCCG
8751 CTTCAGTGAC AACGTCGAGC ACAGCTGCGC AAGGAACGCC CGTCGTGGCC
8801 AGCCACGATA GCCGCGCTGC CTCGTCCTGC AGTTCATTCA GGGCACCGGA
8851 CAGGTCGGTC TTGACAAAAA GAACCGGGCG CCCCTGCGCT GACAGCCGGA
8901 ACACGGCGGC ATCAGAGCAG CCGATTGTCT GTTGTGCCCA GTCATAGCCG
8951 AATAGCCTCT CCACCCAAGC GGCCGGAGAA CCTGCGTGCA ATCCATCTTG

```
9001  TTCAATCATG CGAAACGATC CTCATCCTGT CTCTTGATCA GATCTTGATC
9051  CCCTGCGCCA TCAGATCCTT GGCGGCAAGA AAGCCATCCA GTTTACTTTG
9101  CAGGGCTTCC CAACCTTACC AGAGGGCGCC CCAGCTGGCA ATTCCGGTTC
9151  GCTTGCTGTC CATAAAACCG CCCAGTCTAG CTATCGCCAT GTAAGCCCAC
9201  TGCAAGCTAC CTGCTTTCTC TTTGCGCTTG CGTTTTCCCT TGTCCAGATA
9251  GCCCAGTAGC TGACATTCAT CCGGGGTCAG CACCGTTTCT GCGGACTGGC
9301  TTTCTACGTG TTCCGCTTCC TTTAGCAGCC CTTGCGCCCT GAGTGCTTGC
9351  GGCAGCGTGA AG
```

## Claims

1. An anti-CD45RO/RB humanised antibody comprising a heavy chain variable region of SEQ ID No:9 or of SEQ ID No:10 and a light chain variable region of SEQ ID No:7 or of SEQ ID No:8.

2. An anti-CD45RO/RB humanised antibody of Claim 1 comprising:

   - a heavy chain variable region of SEQ ID No:9 and a light chain variable region of SEQ ID No:7;
   - a heavy chain variable region of SEQ ID No:9 and a light chain variable region of SEQ ID No:8;
   - a heavy chain variable region of SEQ ID No: 10 and a light chain variable region of SEQ ID No:7, or
   - a heavy chain variable region of SEQ ID No: 10 and a light chain variable region of SEQ ID No:8.

3. Isolated polynucleotides encoding a humanised antibody according to any one of claims 1 or 2.

4. Isolated polynucleotides encoding light chain variable region of SEQ ID No:7 or SEG ID No:8 and a heavy chain variable region of SEQ ID No:9 or SEQ ID No:10.

5. A polynucleotide of claim 4 comprising SEQ ID No:11 or SEQ ID No: 12 and SEQ ID No: 13 or SEQ ID No:14.

6. An expression vector comprising polynucleotides according to any of claims 3 to 5.

7. An expression system comprising a polynucleotide according to any one of claims 3 to 5, wherein said expression system is capable of producing an antibody of any one of claims 1 or 2, when said expression system or part thereof is present in a compatible host cell.

8. An isolated host cell which comprises an expression system according to claim 7.

9. Use of the humanised antibody according to any one of claims 1 or 2 as a pharmaceutical.

10. Use of the humanised antibody according to any one of claims 1 or 2, for the preparation of a medicament to be used in the treatment and/or prophylaxis of autoimmune diseases, transplant rejection, psoriasis, inflammatory bowel disease and allergies.

11. A pharmaceutical composition comprising the humanised antibody according to any one of claims 1 or 2 in association with at least one pharmaceutically acceptable carrier or diluent.

## Patentansprüche

1. Humanisierter Anti-CD45RO/RB-Antikörper, umfassend einen variablen Bereich der schweren Kette gemäß SEQ ID Nr.:9 oder SEQ ID Nr.:10 und einen variablen Bereich der leichten Kette gemäß SEQ ID Nr.:7 oder SEQ ID Nr.:8.

2. Humanisierter Anti-CD45RO/RB-Antikörper gemäß Anspruch 1, umfassend:

- einen variablen Bereich der schweren Kette gemäß SEQ ID Nr.:9 und einen variablen Bereich der leichten Kette gemäß SEQ ID Nr.:7;
- einen variablen Bereich der schweren Kette gemäß SEQ ID Nr.:9 und einen variablen Bereich der leichten Kette gemäß SEQ ID Nr.:8;
- einen variablen Bereich der schweren Kette gemäß SEQ ID Nr.:10 und einen variablen Bereich der leichten Kette gemäß SEQ ID Nr.:7 oder
- einen variablen Bereich der schweren Kette gemäß SEQ ID Nr.:10 und einen variablen Bereich der leichten Kette gemäß SEQ ID Nr.:8.

3. Isolierte Polynukleotide, codierend für einen humanisierten Antikörper nach Anspruch 1 oder 2.

4. Isolierte Polynukleotide, codierend für einen variablen Bereich der leichten Kette gemäß SEQ ID Nr.:7 oder SEQ ID Nr.:8 und einen variablen Bereich der schweren Kette gemäß SEQ ID Nr.:9 oder SEQ ID Nr.:10.

5. Polynukleotid gemäß Anspruch 4, umfassend SEQ ID Nr.:11 oder SEQ ID Nr.:12 und SEQ ID Nr.:13 oder SEQ ID Nr.:14.

6. Expressionsvektor, umfassend Polynukleotide nach einem der Ansprüche 3 bis 5.

7. Expressionssystem, umfassend ein Polynukleotid nach einem der Ansprüche 3 bis 5, wobei das Expressionssystem in der Lage ist, einen Antikörper gemäß Anspruch 1 oder 2 zu produzieren, wobei das Expressionssystem oder ein Teil davon in einer kompatiblen Wirtszelle vorliegt.

8. Isolierte Wirtszelle, die ein Expressionssystem nach Anspruch 7 umfasst.

9. Verwendung des humanisierten Antikörpers nach Anspruch 1 oder 2 als Pharmazeutikum.

10. Verwendung des humanisierten Antikörpers nach Anspruch 1 oder 2 zur Herstellung eines bei der Behandlung und/oder Vorbeugung von Autoimmunkrankheiten, Transplantatabstoßung, Psoriasis, entzündlicher Darmerkrankung und Allergien zu verwendenden Arzneimittels.

11. Pharmazeutische Zusammensetzung, umfassend den humanisierten Antikörper nach Anspruch 1 oder 2 in Assoziation mit wenigstens einem pharmazeutisch unbedenklichen Träger oder Verdünnungsmittel.

## Revendications

1. Anticorps anti-CD45RO/RB humanisé, comprenant une région variable de la chaîne lourde ayant la séquence SEQ ID N° 9 ou SEQ ID N° 10 et une région variable de la chaîne légère ayant la séquence SEQ ID N° 7 ou SEQ ID N° 8.

2. Anticorps anti-CD45RO/RB humanisé de la revendication 1, comprenant :

- une région variable de la chaîne lourde ayant la séquence SEQ ID N° 9 et une région variable de la chaîne légère ayant la séquence SEQ ID N° 7 ;
- une région variable de la chaîne lourde ayant la séquence SEQ ID N° 9 et une région variable de la chaîne légère ayant la séquence SEQ ID N° 8 ;
- une région variable de la chaîne lourde ayant la séquence SEQ ID N° 10 et une région variable de la chaîne légère ayant la séquence SEQ ID N° 7, ou
- une région variable de la chaîne lourde ayant la séquence SEQ ID N° 10 et une région variable de la chaîne légère ayant la séquence SEQ ID N° 8.

3. Polynucléotides isolés codant pour un anticorps humanisé selon l'une quelconque des revendications 1 ou 2.

4. Polynucléotides isolés codant pour la région variable de la chaîne légère ayant la séquence SEQ ID N° 7 ou SEQ ID N° 8 et une région variable de la chaîne lourde ayant la séquence SEQ ID N° 9 ou SEQ ID N° 10.

5. Polynucléotide de la revendication 4, comprenant les séquences SEQ ID N° 11 ou SEQ ID N° 12 et SEQ ID N° 13 ou SEQ ID N° 14.

6. Vecteur d'expression comprenant les polynucléotides selon l'une quelconque des revendications 3 à 5.

7. Système d'expression comprenant un polynucléotide selon l'une quelconque des revendications 3 à 5, ledit système d'expression étant capable de produire un anticorps de l'une quelconque des revendications 1 ou 2 quand ledit système d'expression ou une partie de ce dernier est présent dans une cellule hôte compatible.

8. Cellule hôte isolée qui comprend un système d'expression selon la revendication 7.

9. Utilisation d'un anticorps humanisé selon l'une quelconque des revendications 1 ou 2 en tant que produit pharmaceutique.

10. Utilisation de l'anticorps humanisé selon l'une quelconque des revendications 1 ou 2 pour la préparation d'un médicament devant être utilisé dans le traitement et/ou la prophylaxie des maladies autoimmunes, le rejet de transplantation, le psoriasis, l'affection abdominale inflammatoire et les allergies.

11. Composition pharmaceutique comprenant l'anticorps humanisé selon l'une quelconque des revendications 1 ou 2 en association avec au moins un support ou diluant pharmaceutiquement acceptable.

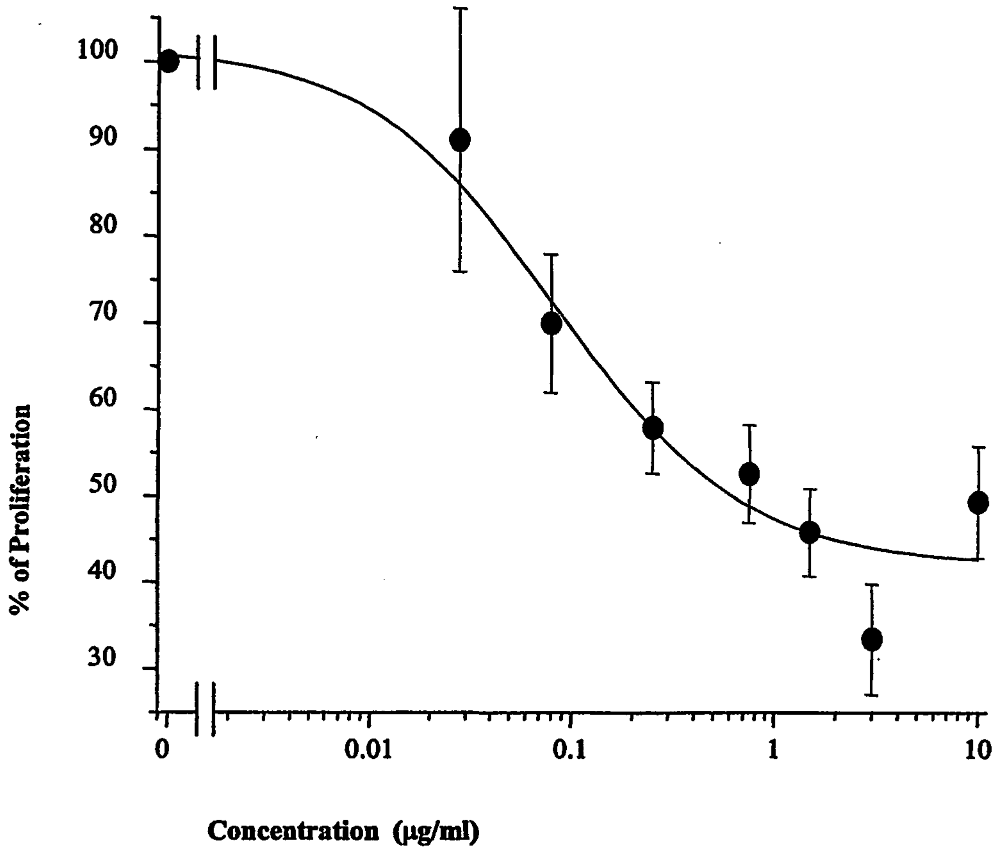
100
90
80
70
60
50
40
30
% of Proliferation
0
0.01
0.1
1
10
Concentration (μg/ml)

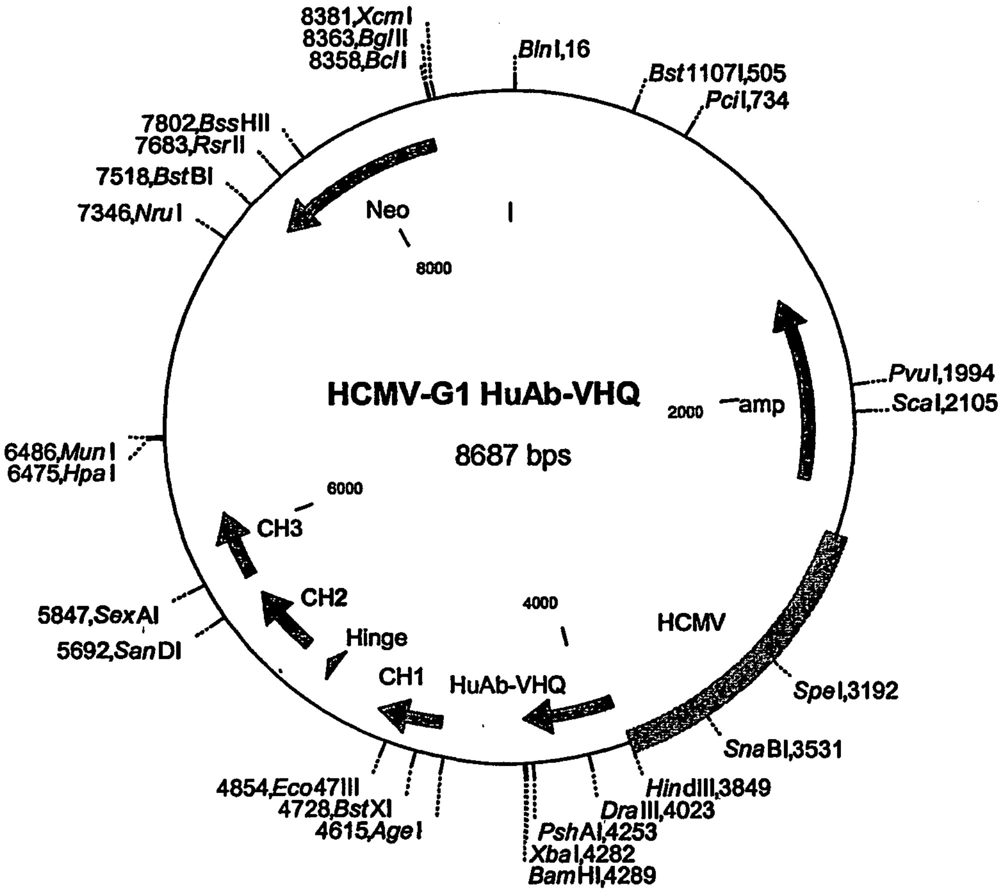
HCMV-G1 HuAb-VHQ
8687 bps
8381,XcmI
8363,BglII
8358,BclI
BlnI,16
Bst1107I,505
PciI,734
7802,BssHII
7683,RsrII
7518,BstBI
7346,NruI
Neo
8000
PvuI,1994
ScaI,2105
2000
amp
6486,MunI
6475,HpaI
6000
CH3
CH2
Hinge
CH1
HuAb-VHQ
4000
HCMV
5847,SexAI
5692,SanDI
SpeI,3192
SnaBI,3531
HindIII,3849
DraIII,4023
PshAI,4253
XbaI,4282
BamHI,4289
4854,Eco47III
4728,BstXI
4615,AgeI

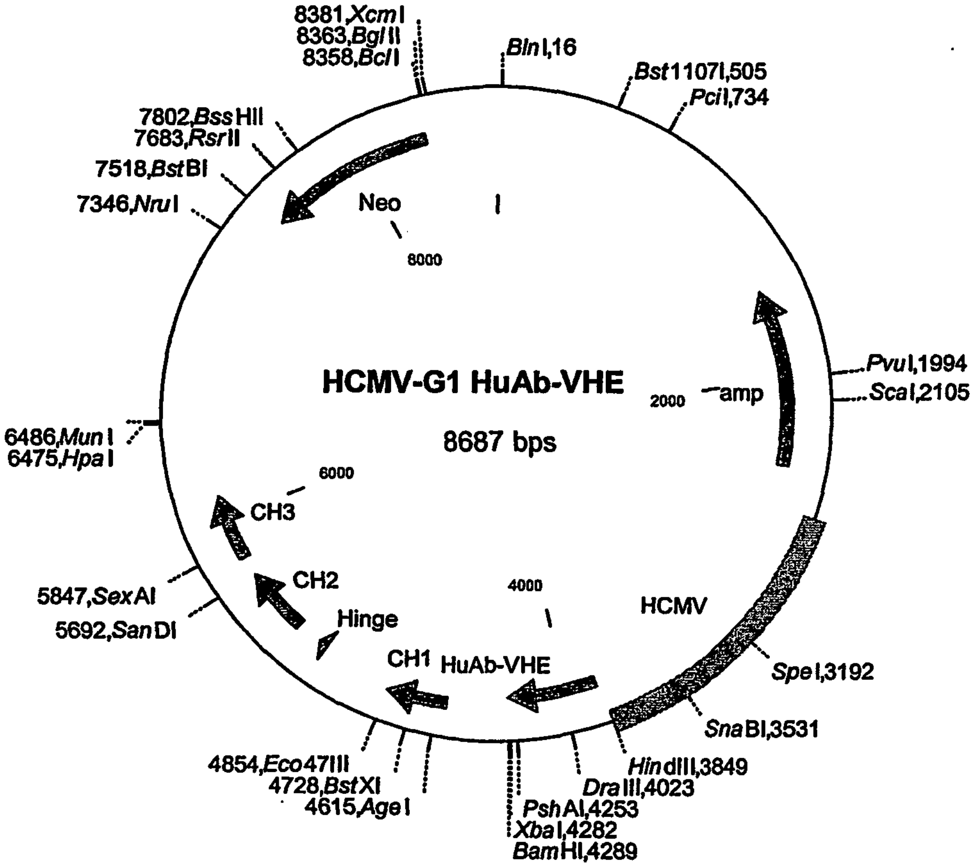
HCMV-G1 HuAb-VHE
8687 bps
8381,XcmI
8363,BglII
8358,BclI
BlnI,16
Bst1107I,505
PciI,734
7802,BssHII
7683,RsrII
7518,BstBI
7346,NruI
Neo
8000
2000
amp
PvuI,1994
ScaI,2105
6486,MunI
6475,HpaI
6000
CH3
CH2
Hinge
CH1
HuAb-VHE
4000
HCMV
5847,SexAI
5692,SanDI
SpeI,3192
SnaBI,3531
HindIII,3849
DraIII,4023
PshAI,4253
XbaI,4282
BamHI,4289
4854,Eco47III
4728,BstXI
4615,AgeI

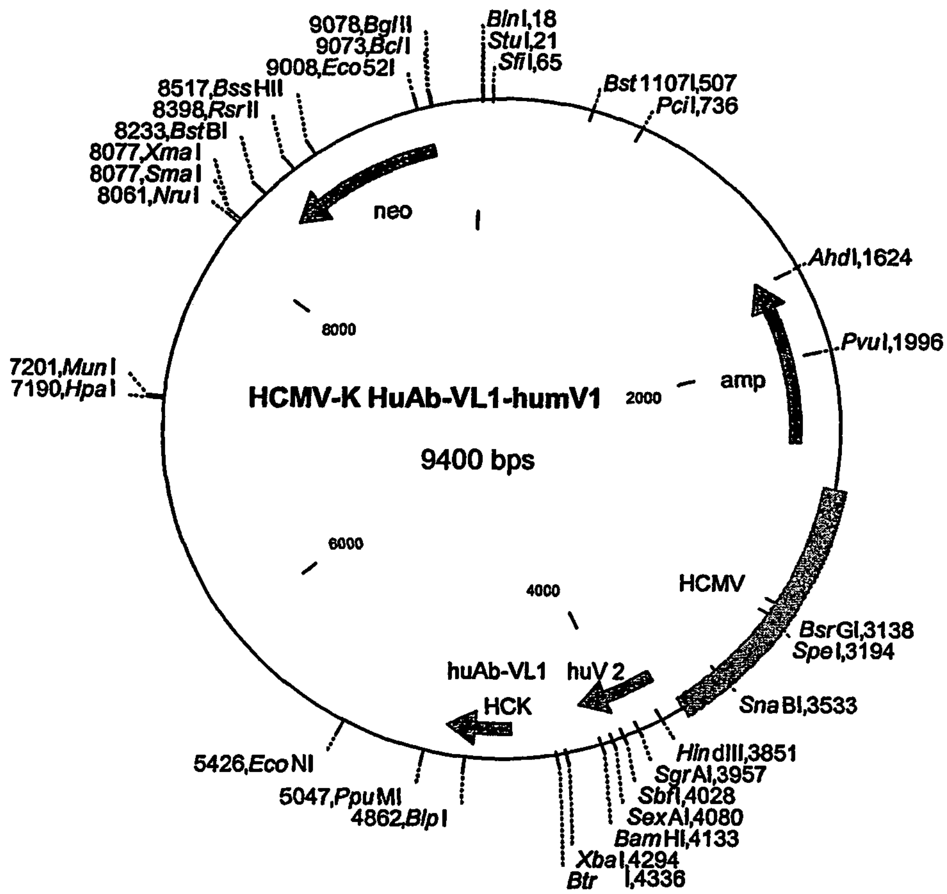
HCMV-K HuAb-VL1-humV1
9400 bps
BlnI,18
StuI,21
SfiI,65
Bst1107I,507
PciI,736
AhdI,1624
PvuI,1996
amp
HCMV
BsrGI,3138
SpeI,3194
SnaBI,3533
HindIII,3851
SgrAI,3957
SbfI,4028
SexAI,4080
BamHI,4133
XbaI,4294
Btr I,4336
huAb-VL1
huV 2
HCK
4862,BlpI
5047,PpuMI
5426,EcoNI
7190,HpaI
7201,MunI
8061,NruI
8077,SmaI
8077,XmaI
8233,BstBI
8398,RsrII
8517,BssHII
9008,Eco52I
9073,BclI
9078,BglII
neo
2000
4000
6000
8000

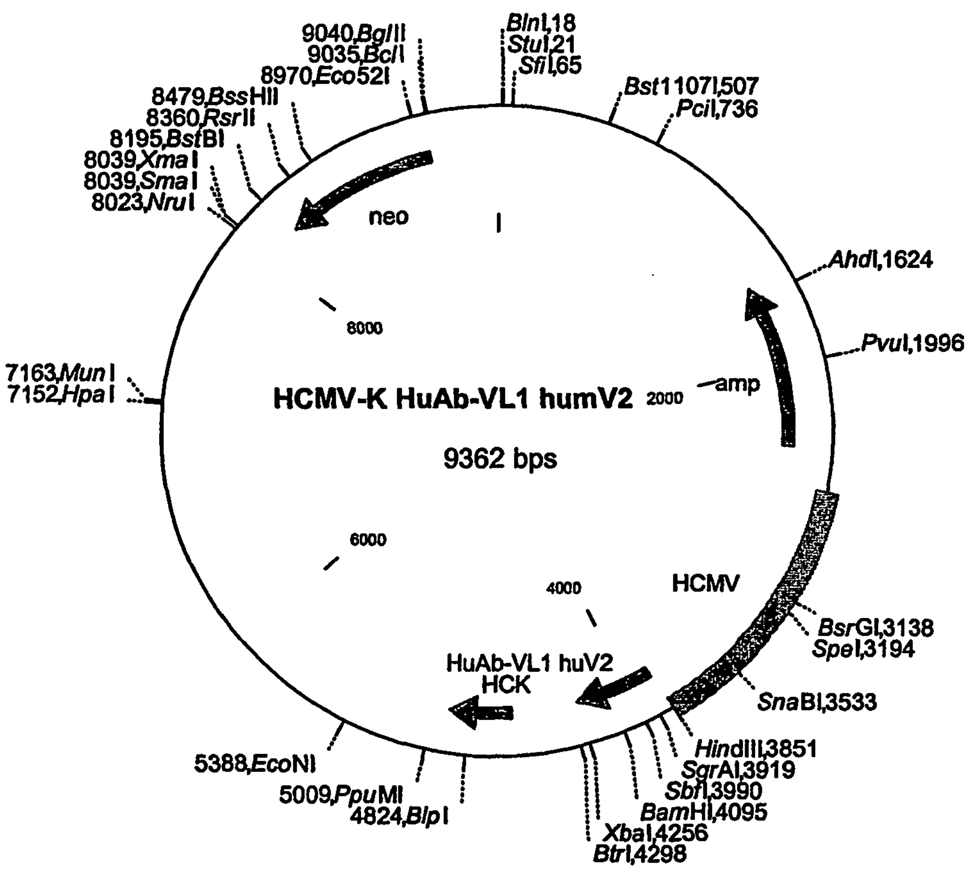
HCMV-K HuAb-VL1 humV2
9362 bps
BlnI,18
StuI,21
SfiI,65
Bst1107I,507
PciI,736
AhdI,1624
PvuI,1996
BsrGI,3138
SpeI,3194
SnaBI,3533
HindIII,3851
SgrAI,3919
SbfI,3990
BamHI,4095
XbaI,4256
BtrI,4298
4824,BlpI
5009,PpuMI
5388,EcoNI
7152,HpaI
7163,MunI
8023,NruI
8039,SmaI
8039,XmaI
8195,BstBI
8360,RsrII
8479,BssHII
8970,Eco52I
9035,BclI
9040,BglII
neo
amp
HCMV
HuAb-VL1 huV2
HCK
2000
4000
6000
8000

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9811918 A
- EP 239400 A


### Non-patent literature cited in the description


- **Trowbridge IS et al.** *Annu Rev Immunol.,* 1994, vol. 12, 85-116
- **Streuli MF. et al.** *J. Exp. Med.,* 1987, vol. 166, 1548-1566
- **Thomas ML. et al.** *Immunol. Today,* 1988, vol. 9, 320-325
- **Beverley PCL et al.** *Immunol.,* 1988, vol. I, 3-5
- **Terry LA et al.** *Immunol.,* 1988, vol. 64, 331-336
- **Aversa G.G et al.** *Transplantation proceedings,* 1989, vol. 21 (1), 349-350
- **Aversa et al.** *Cellular Immunology,* 1994, vol. 158, 314-328
- **T. E. Creighton.** Proteins: Structure and Molecular Properties. W. H. Freeman & Co, 1983
- **Kolbinger et al.** *Protein Eng.,* November 1993, vol. 6 (8), 971-80